(19) Europäisches Patentamt
European Patent Office
Office européen des brevets

(11) **EP 1 944 016 A1**

(12) **EUROPEAN PATENT APPLICATION**

(43) Date of publication:
**16.07.2008 Bulletin 2008/29**

(51) Int Cl.:
**A61K 9/00** (2006.01)   **A61K 9/16** (2006.01)
**A61K 38/28** (2006.01)

(21) Application number: **08075148.0**

(22) Date of filing: **16.08.2002**

(84) Designated Contracting States:
**AT BE BG CH CY CZ DE DK EE ES FI FR GB GR IE IT LI LU MC NL PT SE SK TR**

(30) Priority: **16.08.2001 US 312894 P**

(62) Document number(s) of the earlier application(s) in accordance with Art. 76 EPC:
**02761411.4 / 1 418 890**

(71) Applicants:
• **Baxter International Inc.**
**Deerfield, IL 60015 (US)**
• **Baxter Healthcare SA**
**8304 Wallisellen (CH)**

(72) Inventors:
• **Brown, Larry R.**
**Newton, MA 02159 (US)**

• **Scott, Terrence L.**
**Winchester, MA 01890 (US)**
• **Rashba-Step, Julia**
**Newton, MA 02459 (US)**
• **McGeehan, John K.**
**Woodbury, NJ 08096 (US)**

(74) Representative: **Probert, Gareth David**
**Potter Clarkson LLP**
**Park View House**
**58 The Ropewalk**
**Nottingham NG1 5DD (GB)**

Remarks:
This application was filed on 28-02-2008 as a divisional application to the application mentioned under INID code 62.

(54) **Propellant-based microparticle formulatons**

(57)    A composition comprising:
(a) a plurality of microparticles, said microparticles containing a protein; and
(b) a propellant;

wherein the composition has a fine particle fraction for a particle size of 4.7 μm or less in the range of 25% to 100%, wherein the fine particle fraction is determined using an Andersen Cascade Impactor. There is also disclosed a composition comprising:
a plurality of microparticles, said microparticles containing a protein;

wherein the composition has a fine particle fraction for a particle size of 4.4 μm or less in the range of 25% to 100%, wherein the fine particle fraction is determined using an Andersen Cascade Impactor.

Fig. 1

**Description**

**Field of the Invention**

This invention relates generally to compositions of microparticles, including compositions of microparticles such as microspheres in a propellant, such as a hydrofluoroalkane (HFA) propellant or microspheres for delivery by dry powder inhalation methods, and methods for making and using said compositions.

**Background of the Invention**

The preparation and delivery of therapeutic proteins of interest is an area of concentrated research and development activity in the pharmaceutical industry. It is highly desirable to formulate proteins with select release characteristics in the patient with maximum clinical effectiveness and ease of manufacture. For pulmonary administration, the protein is ideally prepared in the form of discrete microspheres, which are solid or semi-solid particles having a diameter of between 0.5 and 5.0 microns. It is also desirable for the particles to have a protein content as high as possible for maximum therapeutic effectiveness, as well as to eliminate non-therapeutic excipients.

Microspheres have been commercially available for biochemical and biotherapeutic applications for many years. For example, antibodies conjugated to beads produce relatively large particles which are specific for a particular ligand. These large antibody-coated particles are used to bind receptors on the surface of a cell for cellular activation, for binding to a solid phase for immunoaffinity purification, and for the delivery of therapeutic agents to a target using tissue or tumor-specific antibodies. The beads can be formed from synthetic polymers or proteins, although synthetic polymers are sometimes preferred due to durability and cost.

Microparticles produced by standard production methods frequently have a wide particle size distribution, lack uniformity, fail to provide adequate release kinetics, and are difficult and expensive to produce. Frequently, the polymers used to prepare these microspheres are primarily soluble in organic solvents, requiring the use of special facilities designed to handle organic solvents. The organic solvents can denature proteins or peptides contained in the microspheres, and may also be toxic to the environment, present an inflammatory hazard, as well as being potentially toxic when administered to humans or animals. In addition, the microparticles may be large and tend to form aggregates, requiring a size selection process to remove particles considered to be too large for administration to patients by injection or inhalation. This requires sieving and resulting product loss.

U.S. Patent No. 5,981,719, U.S. Patent No. 5,849,884 and U.S. Patent No. 6,090,925, the disclosures of which are incorporated by reference herein in their entirety, describe microspheres formed by combining a macromolecule, such as a protein or peptide, and a polymer in an aqueous solution at a pH at or near the isoelectric point of the protein. The solution is heated to prepare microspheres having a protein content of greater than 40%. The microspheres thus formed comprise a matrix of substantially homogeneous proteins and varying amounts of polymers, which permit the aqueous medium to enter and solubilize the components of the microspheres. The microspheres can be designed to exhibit short-term or long-term release kinetics, providing either rapid or sustained release characteristics.

U.S. Patent No. 6,051,256 relates to processes for preparing powders of biological proteins by atomizing liquid solutions of the proteins, drying the droplets, and collecting the resulting particles. Biological proteins which reportedly can be used in this process include insulin and calcitonin.

It will be appreciated that there is a continuing need for a process for preparing and delivering biological agents as microspheres to maximize their effectiveness and optimize the dosage of the therapeutic agent.

Therefore, there is an on-going need for development of new and superior methods for making microspheres that are useful for therapeutic and diagnostic applications and in particular, that are useful in making and using pulmonary formulations. Preferably, such methods and improved formulations would permit a cost effective production method and the release of active agents in a predictable, consistent manner.

**Summary of the Invention**

The invention solves these and other problems by providing methods and compositions for making and using microparticles, e.g., microspheres, containing protein or peptide therapeutic and/or diagnostic agents *in* vivo and *in vitro.* The microspheres are particularly useful as active therapeutic components of inhalation devices for pulmonary administration to human patients.

There are at least four areas where the present invention is able to address the limitations in this field: a) formation of inherently stable protein microspheres, b) forming these microspheres in a particle size range capable of reaching the deep lung, c) formulating the microspheres such that they suspend efficiently and homogeneously in HFA propellant so that they can be delivered from a MDI or a MDI-type device in a particle size range distribution that does not aggregate and that one would expect could reach the deep lung, and d) formulating the microspheres so that they remain biochemically stable in MDI or MDI-type devices and propellants.

According to a first aspect of the invention, a composition including: a plurality of microparticles (e.g., microspheres), said microparticles containing a protein or a polypeptide; and a propellant (e.g., a hydrofluoroalkane (HFA) propellant) is provided. The composition has a "fine particle fraction" (FPF) in the range of 25% to 100%. "Fine particle fraction" (previously referred to as "respirable fraction") is a term of art which is defined as follows:

"Fine particle fraction" (FPF) refers to the total amount of the drug deposited on the stages in the Andersen cascade impaction studies, within an appropriate particle size range for the drug being tested, divided by the amount total drug delivered from the mouthpiece of the inhaler into the impactor.

The FPF for an MDI and a particle which is less than or equal to 4.7 $\mu$m; the FPF for a DPI and a particle which is less than or equal to 4.4 $\mu$m:

Dry Powder Inhaler: for DPI (60 1pm) a particle size range of $\leq$ 4.4 $\mu$m
Dry Powder Fine Particle Fraction (4.4) is defined as the percentage of the sum of the mass of particles less than or equal to 4.4 microns in diameter divided by the total emitted dose from the device and the mouthpiece of the device.

$$FPF = \frac{\sum Particle\ Mass \leq 4.4\mu m}{\sum particle\ mass\ in\ all\ the\ stages\ plus\ mouthpiece} \times 100$$

Metered Dose Inhaler: for MDI (28.3 lpm) a particle size range $\leq$ 4.7 $\mu$m
Metered Dose Inhaler Fine Particle Fraction (4.7) is defined as the percentage of the sum of the mass of particles less than or equal to 4.7 microns in diameter divided by the total emitted dose from the device and the mouthpiece of the device.

$$FPF = \frac{\sum Particle\ Mass \leq 4.7\mu m}{\sum particle\ mass\ in\ all\ the\ stages\ plus\ mouthpiece} \times 100$$

According to convention, the FPF is expressed as a percentage. (See, RSP 24/NF19, United States Pharmacopeial convention, Rockville, MD, pages 1895-1912, January, 2000. This reference also describes the use of the Andersen Cascade Impactor (apparatus 1) to determine FPF values.) The terms $\mu$, $\mu$m, and micron(s) are used interchangeably herein.

Biocompatible microparticles that can be used in accordance with the methods of the invention are selected as described herein to have a size, a density and physical chemical properties to result in a propellant-microparticle (e.g., HFA-microsphere) formulation having a fine particle fraction in the range of 25% to 100%. In general, microparticles such as microspheres that are useful for pulmonary delivery of a therapeutic protein or peptide to the lung have a diameter in the range of about 0.2 $\mu$ to about 10$\mu$; and a density in the same approximate range as the density of the propellant, e.g., about 0.5 gm/cc to about 1.6 gm/cc. Preferably, the microparticles are microspheres that contain at least 40% (w/w) protein. More preferably, the microspheres are dispersed in a hydrofluoroalkane propellant. Exemplary hydrofluoroalkane propellants are HFA P134a and HFA P227.

Preferably, the microparticles are microspheres that have a narrow particle size distribution, e.g., at least 90 % of the microspheres having a diameter in the range of about 0.1 to about 10 $\mu$. More preferably, at least 95 % of the microspheres have a diameter in the range of about 0.1 to about 10 $\mu$ (more preferably, in the range of about 0.1 to about 5 $\mu$; most preferably, in the range of about 0.1 to about 3$\mu$); Most preferably, at least 99 % of the microspheres have a diameter in the range of about 0.1 to about 10 $\mu$ (more preferably, in the range of about 0.1 to about 5 $\mu$; most preferably, in the range of about 0.1 to about 3$\mu$).

In the particularly preferred embodiments, the microparticles are microspheres that are formed using polyethylene glycol, polyvinyl pyrrolidone, or a combination, or copolymer thereof. In yet other particularly preferred embodiments, the microspheres are formed of albumin and other components (e.g., dextran sulfate, hetastarch). The albumin microspheres are particularly useful for carrying low molecular weight molecules.

The microspheres contain a therapeutic or diagnostic protein or peptide. For ease of discussion, the term "protein" as used herein is meant to embrace peptide. The preferred proteins for use in accordance with the methods of the invention include insulin, parathyroid hormone, human growth hormone, interferon, GCSF, calcitonin, leuprolide acetate, and any of the therapeutic molecules identified in Table 1. Other proteins that can be used in accordance with the compositions and methods of the invention are provided in the detailed

description of the invention.

In some preferred embodiments, the compositions of this and other aspects of the invention do not contain a surfactant. In these and other embodiments, the microparticles, particularly microspheres, remain in suspension for a period of time of at least 10 seconds, preferably, at least 20, 30, 40, 50 seconds, at least 2, 5, 10, 30 minutes, at least 1, 2, 5, 10 hours, at least 1, 2, 3, 4, 5, 6, 7, 14, 28 days, following agitation.

It is to be understood that any of the optional limitations which represent the preferred embodiments of the first aspect of the invention are applicable to other aspects of the invention. Thus, the invention provides compositions and methods in which any one or more limitations which represent a preferred embodiment of the invention can be used alone or in combination with another aspect of the invention.

According to a second aspect of the invention, a composition comprising: a plurality of microparticles (preferably, microspheres), said microparticles containing a protein or polypeptide; and a propellant such as a hydrofluoroalkane (HFA) propellant, is provided; however, in this aspect, the composition does not contain a surfactant. Preferably, the composition has a fine particle fraction in the range of 25% to 100%.

According to a third aspect of the invention, a method for preparing a pulmonary preparation is provided. The method involves: 1) selecting a propellant, such as a hydrofluoroalkane propellant having a known density, $\rho_{propellant}$ (e.g., $\rho_{hydrofluoroalkane}$); 2) selecting a microparticle (e.g., microsphere) having a microparticle density $\rho_{microparticle}$ (e.g., $\rho_{microsphere}$) such that the ratio of $\rho_{microparticle}$ to $\rho_{propellant}$ is in the range of .05 to 30 and, more preferably, in the range of 0.5 to 3.0; and 3) contacting a plurality of the microparticles with the propellant to form the pulmonary preparation. Preferably, the propellant is an HFA propellant such as HFA P134a, HFA P227, or a blend of these propellants. In these and other embodiments, the composition preferably does not include a surfactant.

According to a fourth aspect of the invention, a method of administering a protein to the pulmonary system of a subject is provided (e.g., via a metered dose or dry powder inhaler or other inhalation device). The method involves administering to the respiratory tract of a subject in need of treatment, an effective amount of a composition of the invention to treat the condition. In certain embodiments, the composition of the first aspect of the invention is administered; in yet other embodiments, the composition of the second aspect of the invention is administered.

According to a fifth aspect of the invention, a method of manufacture is provided. The method involves dispersing one or more therapeutic doses into a pulmonary delivery device, each of said therapeutic doses containing a therapeutically effective amount of a composition of the invention.

According to a sixth aspect of the invention, a package containing a container (e.g., capsule, canister) containing one or more therapeutic doses of a composition of the invention is provided. The package preferably provides instructions for using the container to deliver its contents to a pulmonary delivery device and, thereafter, deliver a therapeutically effective dose of the microspheres to a patient.

According to a seventh aspect of the invention, an inhalant delivery device containing one, two, or more therapeutic doses containing a therapeutically effective amount of the microparticles of the invention is provided. Exemplary devices include dry powder inhalers, metered dose inhalers, and other inhalation devices.

The use of the foregoing compounds and compositions for the manufacture of a medicament is provided herein.

These and other aspects of the invention will be described in greater detail below. Throughout this disclosure, all technical and scientific terms have the same meaning as commonly understood by one of ordinary skill in the art to which this invention pertains unless defined otherwise.

**Brief Description of the Drawings**

Figure 1 shows particle size determined by laser light scattering Coutler LS230. 95% of the Insulin microspheres are between 0.95 and 1.20 microns.
Figure 2 depicts aerodynamic diameter determined using a TSI Aerosizer (Model 322500, St. Paul, MN.)
Figure 3 shows Andersen Cascade Impactor studies with 10 mg insulin delivered from Aerolizer DPI (JM032701C).
Figure 4 shows *in vitro* Andersen cascade impaction studies with insulin delivered from vials containing HFA P134a and HFA P227.
Figure 5 depicts glucose depression after SC injections of Insulin microspheres in SC rats.

Figure 6 depicts glucose depression after intratracheal instillation of Insulin microspheres.
Figure 7 compares suspension stability.
Figure 8 shows TC-99m insulin lung distribution in dog lung.
Figure 9 shows an assay for content and related substances (USP).
Figure 10 is a comparison of MDI activity 1 week and 4 months after the fill.
Figure 11 depicts insulin microsphere administration to dog via DPI.
Figure 12 shows percent emitted dose of Insulin microspheres from MDI.
Figure 13 depicts insulin stability in HFA P 134a.

## Detailed Description of the Invention

The invention provides methods and compositions for making and using microparticles, such as microspheres, containing therapeutic and/or diagnostic agents *in vivo* and *in vitro.* The compositions are particularly useful as pulmonary formulations. In contrast to the prior art formulations, the propellant (e.g., hydrofluoroalkane) microparticle formulations disclosed herein exhibit surprising and unexpected improved properties as pulmonary formulations. In particular, the Examples illustrate the properties of exemplary HFA-microsphere (containing insulin) formulations that were prepared in accordance with the methods disclosed herein. In contrast to unsuccessful prior art efforts to prepare pulmonary formulations containing proteins, the results disclosed herein evidence that the compositions of the invention exhibited surprising and advantageous fine particle fraction values, and the ability to remain in suspension for a suitable time period. One skilled in the art will appreciate that stable suspensions are preferable for ensuring uniformity of dosing using any inhalant delivery device. The protein microspheres described herein also exhibit improved and unexpected stability in the presence of propellants, both freon-based and freon substitutes, commonly used in inhalation devices for pulmonary delivery. Thus, the invention advantageously provides compositions that exhibit significantly improved characteristics compared to pulmonary protein formulations suggested in the prior art, especially those that have relied on surfactant and emulsion methods to incorporate drugs. In addition, the microspheres of the invention can be prepared without the need for spray drying or milling processes.

According to a first aspect of the invention, a composition including: a plurality of microparticles (e.g., microspheres), said microparticles containing a protein or polypeptide (collectively referred to as "protein"); and a propellant (e.g., a hydrofluoroalkane (HFA) propellant), is provided. The composition has a fine particle fraction in the range of 25% to 100%. Biocompatible microparticles (e.g., microspheres) that can be used in accordance with the methods of the invention, are those which have a size and a density to result in a pulmonary formulation having a fine particle fraction in the range of 25% to 100%. In particularly preferred embodiments, the microparticles are microspheres which have a protein content which is in the range of 20 to 100% of the total weight of the microsphere. In general, microparticles (e.g., microspheres) that are useful for pulmonary delivery of a therapeutic protein or peptide to the lung have a diameter in the range of about 0.1 $\mu$ to about 10$\mu$ (for some applications, 0.1 $\mu$ to 5$\mu$; and for other applications, 0.1 $\mu$ to 3$\mu$); and a density in the range of about 0.6 gm/cc to about 2.5 gm/cc (more preferably, 0.6 gm/cc to 1.8 gm/cc; and most preferably, 1.2 gm/cc to 1.7 gm/cc). In some preferred embodiments, the microspheres have a protein content that is at least 40% of the microsphere weight (more preferably, at least 50%, 60%, 70%, or 80%; and most preferably, at least 90%, 95% or 100%).

As used herein, the term, "microparticles" refers to microparticles, microspheres, and microcapsules, that are solid or semi-solid particles having a geometric or aerodynamic diameter of less than 100 microns, more preferably less than 10 microns, which can be formed of a variety of materials, including synthetic polymers, proteins, and polysaccharides. A number of different techniques are routinely used to make these microparticles from synthetic polymers, natural polymers, proteins and polysaccharides, including phase separation, solvent evaporation, emulsification, and spray drying. Exemplary polymers used for the formation of microspheres include homopolymers and copolymers of lactic acid and glycolic acid (PLGA) as described in U.S. Pat. No. 5,213,812 to Ruiz, U.S. Pat. No. 5,417,986 to Reid et al., U.S. Pat. No. 4,530,840 to Tice et al., U.S. Pat. No. 4,897,268 to Tice et al., U.S. Pat. No. 5,075,109 to Tice et al., U.S. Pat. No. 5,102,872 to Singh et al., U.S. Pat. No. 5,384,133 to Boyes et al., U.S. Pat. No. 5,360,610 to Tice et al., and European Patent Application Publication Number 248,531 to Southern Research Institute; block copolymers such as tetronic 908 and poloxamer 407 as described in U.S. Pat. No. 4,904,479 to Illum; and polyphosphazenes as described in U.S. Pat. No. 5,149,543 to Cohen et al.

As used herein, the term, "microspheres", refers to microparticles that are substantially spherical in shape and that have dimensions generally of between about 0.1 microns and 10.0 microns in diameter. The microspheres disclosed herein typically exhibit a narrow size distribution, and are formed as discrete particles. Illustrative methods for forming microspheres are described below.

In a preferred embodiment, the microspheres are formed by mixing an aqueous or aqueous-miscible polymer solution and an aqueous protein solution and applying an energy source, such as heat, to form the microspheres. (See, e.g., the Examples.) In general, such processes involve heating these solutions to a temperature in the range of from about 37°C to about 95°C for a time period of about 1 minute to about 24 hours. As used herein, an "aqueous solution", refers to solutions of water alone, or water mixed with one or more water-miscible solvents, such as ethanol, DMSO, acetone N-methyl pyrrolidone, and 2-pyrrolidone; however, the preferred aqueous solutions do not contain detectable organic solvents.

Preferably, the polymer is selected from the group consisting of carbohydrate-based polymers, polyaliphatic alcohols, poly(vinyl) polymers, polyacrylic acids, polyorganic acids, polyamino acids, polyethers, naturally occurring polymers, polyimids, polyesters, polyaldehydes, co-polymers, block co-polymers, tertpolymers, surfactants, branched polymers, cyclo-polymers, and mixtures thereof. More preferably, the polymer is dextran, polyethylene glycol, polyvinyl pyrrolidone, co-polymers of polyethylene glycol and polyvinyl pyrrolidone, polyvinyl alcohol, or co-polymers of polyoxyethylene and polyoxypropylene, and mixtures thereof. Most preferably, the polymer is a co-polymer of polyethylene glycol and polyvinyl pyrrolidone, or a co-polymer of polyoxyethylene and polyoxypropylene.

In the preferred embodiments, the polymer is a water soluble polymer. As used herein, a "water soluble polymer" refers to a polymer or mixture of polymers which, preferably, are capable of causing volume exclusion or macromolecular crowding.

Suitable water soluble polymers for forming pulmonary microspheres include soluble linear or branched polymers, preferably those having a low molecular weight. As used herein in reference to pulmonary microspheres, low molecular weight polymer means polymers having a molecular weight that is suitable to be adequately cleared from the lung. Polymers can be highly water soluble, moderately-water soluble, or slightly water soluble (greater than 2% wt/vol water soluble). The preferred water soluble polymers are water soluble or soluble in a water miscible solvent. The water soluble polymers may be solubilized by first being dissolved in water, an aqueous buffered solution, or a water miscible solvent and then combining the polymer solution with an aqueous solvent. In one embodiment, the water soluble polymer is a carbohydrate-based polymer.

The preferred polymer is polyvinylpyrrolidone, polyethylene glycol, dextran, polyoxyethylene-polyoxypropylene copolymer, polyvinyl alcohol, starch, hetastarch, or mixtures thereof, the characteristics of which are described in more detail below. The polymer or polymer mixture may be prepared in accordance with the methods set forth in U.S. Pat. No. 5,525,519 to James E. Woiszwillo, or PCT Patent Application No. US93/00073 (International Publication No. WO 93/14110), filed Jan. 7, 1993 and published on Jul. 22, 1993 by James E. Woiszwillo, both of which are incorporated herein by reference), in which the polymer is dissolved in water or an aqueous solution, such as a buffer, in a concentration between approximately 1 and 50 g/100 ml depending on the molecular weight of the polymer. The preferred total polymer concentration in the polymer solution is between 5% and 80%, expressed as weight/volume percent. The preferred concentration of each polymer in the polymer solution is between 0.5% and 25%.

Polyoxyethylene-polyoxypropylene copolymer, also known as poloxamer, is sold by BASF (Parsippany, N.J.) and is available in a variety of forms with different relative percentages of polyoxyethylene and polyoxypropylene within the copolymer.

PVP is a non-ionogenic, hydrophilic polymer having a mean molecular weight ranging from approximately 2,000 to 700,000 (preferably, from approximately 2000 to 40,000) and the chemical formula $(C_6H_9NO)[_n]$. PVP is also known as poly[1-(2-oxo-1-pyrrolidinyl)ethylene], Povidone™, Polyvidone™, RP 143™, Kollidon™, Peregal ST™, Periston™, Plasdone™, Plasmosan™, Protagent™, Subtosan™, and Vinisil™. PVP is non-toxic, highly hygroscopic and readily dissolves in water or organic solvents.

Polyethylene glycol (PEG), also known as poly(oxyethylene) glycol, is a condensation polymer of ethylene oxide and water having the general chemical formula $HO(CH_2CH_2O)[n]H$.

Dextran is a term applied to polysaccharides produced by bacteria growing on a sucrose substrate. Native dextrans produced by bacteria such as Leuconostocmesenteroides and *Lactobacteria dextranicum* usually have a high molecular weight. Dextrans are routinely available and are used in injectable form as plasma expanders in humans.

Polyvinyl alcohol (PVA) is a polymer prepared from polyvinyl acetates by replacement of the acetate groups with hydroxyl groups and has the formula $(CH_2CHOH)[n]$. Most polyvinyl alcohols are soluble in water.

PEG, dextran, PVA and PVP are commercially available from chemical suppliers such as the Sigma Chemical Company (St. Louis, MO).

Preferably, the polymer is a polymer mixture containing an aqueous solution of PVP having a molecular weight between 2,000 and 360,000, most preferably 2,000 to 40,000, and PEG having a molecular weight between 200 and 35,000. PVP having a molecular weight of 2,000 and PEG having a molecular weight of 3500 is preferred. Preferably, the PVP is dissolved in buffer and PEG is added to the aqueous PVP solution. The concentration of each polymer is preferably between 0.5 and 50 g/100 ml depending of the molecular weight of each polymer.

An alternative preferred polymer is a dextran, having a molecular weight from approximately 3000 to 500,000 daltons.

Suitable polymers include, in addition to the specific polymers mentioned above, high molecular weight linear or branched chain polymers which are soluble in water, or in a water miscible solvent, or both. Exemplary water soluble polymers which are useful in this invention also are described in U.S. Patent Nos. 6,090,925; 5,981,719; and 5,578,709;

and in pending, commonly assigned U.S. Patent Application No. 09/420,361, filed October 18, 1999, and U.S. Patent Application No. 60/244,098, filed October 27, 2000; the disclosures of which are incorporated herein in their entirety.

In the particularly preferred embodiments, the microspheres are formed using polyethylene glycol, polyvinyl pyrrolidone, or a combination or co-polymer thereof. In yet other particularly preferred embodiments, the microspheres are formed of albumin and other components (e.g., dextran sulfate, hetastarch). The albumin microspheres are particularly useful for carrying low molecular weight peptides.

Exemplary preferred embodiments of microspheres that can be made and used in accordance with the methods and compositions of the invention include those having the following approximate compositions:

A. Protein (40-100%); PEG (1-60%); PVP (1-60%);
B. Protein (40-100%); dextran sulfate (1-60%); hetastarch (0-60%);
C. Albumin (40-90%); leuprolide acetate (3-50%); dextran sulfate (1-60%); hetastarch (0-60%);
D. Protein (e.g., insulin, MW 5700) (95-100%); PEG/PVP (0-5%);
E. Protein (e.g., human serum albumin (HSA)) (30%); dextran sulfate (20%); leuprolide acetate (50%);
F. Protein (e.g., casein) (95-100%); PEG/PVP (0-5%);
G. Protein (e.g., DNAse) (95-100%); PEG/PVP (0-5%);
H. Protein (e.g., horse radish peroxidase, MW 44,000) (95-100%); PEG/PVP (0-5%);
I. Protein (e.g., catalase) (95-100%); PEG/PVP (0-5%);
J. Protein (e.g., lysozyme, MW 14,300) (95-100%); PEG/PVP (0-5%);
K. Protein (e.g., crystalline) (95-100%); PEG/PVP (0-5%);
L. Protein (thyroglobulin, MW 670,000) (95-100%); PEG/PVP (0-5%);
M. Protein (ribonuclease A, MW 13,700) (95-100%); PEG/PVP (0-5%).

Additional exemplary proteins that can be incorporated into the compositions of the invention are described in more detail below. Thus, for example, the microspheres of the invention that can be made and used in accordance with the methods and compositions of the invention include those having 95-100% of any one or more of the therapeutic proteins of Table 1 and 0-5% PEG/PVP.

The volume of polymer added to the protein varies depending on the size, quantity and concentration of the protein. Preferably, two volumes of the polymer mixture at a 0.5-50% total polymer concentration are added to one volume of a solution containing the protein, typically at a concentration of 0.1 to 250 mg/ml. The polymer is present in a liquid phase during the reaction with protein.

The process can be operated in a batch or continuous mode. Each of these methods is illustrated in the Examples.

The preferred energy source is heat. However, it will be understood by those skilled in the art that other energy sources include radiation, ionization, osmotic forces, and electrical forces, alone or in combination with heat, sonication, vortexing, mixing or stirring. Microsphere formation can occur immediately upon exposure to the energy source or may require an extended exposure to the energy source depending on the characteristics of the components and conditions. Preferably, the protein-polymer solution mixture, is incubated in a water bath at a temperature greater than or equal to 37°C and less than or equal to 99°C for between approximately 1 minute and 24 hours. Most preferably, the mixture is incubated for 5-30 minutes at a temperature between 50°C and 90°C. The maximum incubation temperature is determined by the characteristics of the protein and the ultimate function of the microsphere.

The formed microspheres are separated from the non-incorporated components of the incubation mixture by conventional separation methods well known to those skilled in the art. The incubation mixture may be centrifuged or subject to filtration or diafiltration to separate the microspheres from the soluble non-incorporated components. Alternatively, a suspension containing formed microspheres is filtered so that the microspheres are retained on the filter and the non-incorporated components pass through the filter.

Further purification of the microspheres is achieved by washing in an appropriate volume of a washing solution. The preferred washing solution is water, or a water-miscible solvent capable of removing the water soluble polymers. Repeated washings can be utilized as necessary and the microspheres separated from the wash solution as described above.

As mentioned above, the characteristics of the microspheres can be altered by manipulating the incubation conditions. For example, the release kinetics of the microspheres may be retarded by increasing the reaction temperature or extending the length of reaction time during microsphere formation. Release kinetics are also manipulated by choosing different polymers, different concentrations of polymers, different concentrations of proteins, or different ratios of polymers used in the formation of the microspheres.

Microsphere size, shape and release kinetics can also be controlled by adjusting the microsphere formation conditions. For example, microsphere formation conditions can be optimized to produce smaller or larger microspheres, or the overall incubation time or incubation temperature can be increased, resulting in microspheres which have prolonged release kinetics.

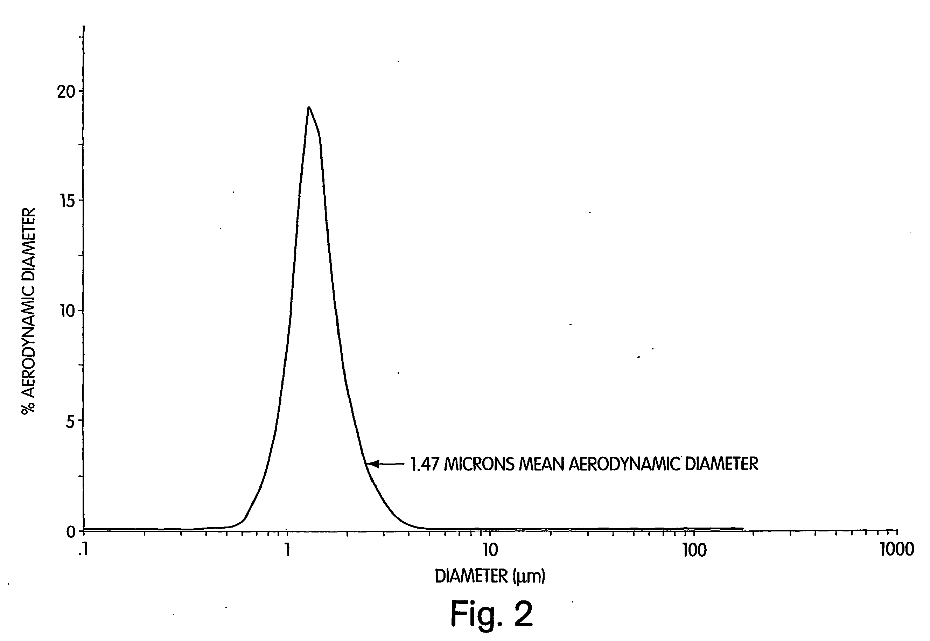

As used herein, a "homogeneous distribution" refers to a population of microspheres wherein >90% of the microspheres have a diameter in the above-cited range. Preferably, the microspheres have a homogeneous distribution with at least 95% of the microspheres having a diameter in the above-cited range. In some embodiments, >95% of the microspheres have a diameter in the range of 0.1 to 10 microns (by number average, surface area average, and volume average), as analyzed by a Coulter laser light diffraction particle size analyzer). Preferably, these populations of microspheres that have a homogeneous distribution show little or no evidence of aggregation. (See, e.g., Figure 2).

The proteins and peptides which are useful in the practice of this invention include both therapeutic and diagnostic agents. In general, the proteins are characterized by the ability to form intact, discrete microspheres having a high content of protein in the presence of an energy source, such as heat, in the presence of the above-noted polymers. Preferably, the protein comprises at least about 90% by weight of the microspheres, more preferably at least about 95% by weight, and most preferably at least about 99%. In especially preferred embodiments, the protein which is released from the microsphere has a structure or function that is indistinguishable from the starting protein.

For ease of discussion, the term "protein" as used herein is meant to embrace peptides.

The protein component of the microsphere may be a carrier protein or a therapeutic protein (see, e.g., Table 1), which represents from about 20 to 100% (wt%) of the microsphere.

As used herein, a "carrier protein" refers to a protein which has a molecular weight of at least about 1500 and which can exist as a three dimensional structure. The carrier protein can also be a therapeutic protein, i.e., a protein which has a therapeutic activity; however, in general, the phrase "carrier protein" will be used in this application to refer to a protein which has a primary function to provide a three dimensional structure, for the purpose of microsphere formation, even if the carrier protein also may have a secondary function as a therapeutic agent. In certain preferred embodiments, the carrier protein is albumin, particularly, human serum albumin. The protein microspheres of the invention, optionally, further include a therapeutic agent such as a steroid (e.g., estradiol, testosterone, prednisolone, dexamethasone, hydrocortisone, lidocaine base, procaine base), or any other such chemical entity known to bind to albumin such as GCSF, or paclitaxel.

In yet other embodiments (discussed below), the microspheres of the invention further include a therapeutic agent (preferably a peptide). In certain other embodiments, the protein that comprises the matrix is a therapeutic protein (e.g., a hormone such as insulin or human growth hormone) and the microsphere is constructed and arranged to provide specified release kinetics of the therapeutic protein in vivo. More preferably, the microsphere is constructed and arranged to provide specified release kinetics of the therapeutic protein in the absence of significant swelling of the microsphere.

Table 1. Proteins

| PROTEINS | |
| --- | --- |
| CARRIER PROTEINS | THERAPEUTIC PROTEINS OR PEPTIDES OR MOLECULES |
| Albumins (preferably, human serum albumin); BSA; IgG; IgM; insulin; hGH; lysozyme; alpha-lactoglobulin; basic fibroblast growth factor; VEGF; chymotrypsin; trypsin; carbonic anhydrase; ovalbumin; phosphorylase b; alkaline phosphatase; beta-galactosidase; fibrinogen; polyaminoacids (e.g., poly-lysine, polyarginine); immunoglobulins (e.g., antibodies); casein; collagen; soy protein; and gelatin. | Insulin; human growth hormone; GCSF; GMCSF; LHRH; VEGF; basic fibroblast growth factor (bFGF); asparaginase; tPA; urokinase; streptokinase; interferon; glucagon; ACTH; oxytocin; secretin; vasopressin; levothyroxin; parathyroid hormone, calcitonin; DNAse, alpha1 anti-trypsin; angiogenesis inhibitors or promoters; somatostatin and analogs; cytokines (e.g., interferon, interleukin); immunoglobulins. |

The preferred physiologically active proteins include peptide hormones, cytokines, growth factors, factors acting on the cardiovascular system, factors acting on the central and peripheral nervous systems, factors acting on humoral electrolytes and hemal substances, factors acting on bone and skeleton, factors acting on the gastrointestinal system, factors acting on the immune system, factors acting on the respiratory system, factors acting on the genital organs, and enzymes.

Exemplary hormones and hormone modulators include insulin, proinsulin, C-peptide of insulin, a mixture of insulin and C-peptide of insulin, hybrid insulin cocrystals (Nature Biotechnology, 20, 800-804, 2002), growth hormone, parathyroid hormone, luteinizing hormone-releasing hormone (LH-RH), adrenocorticotropic hormone (ACTH), amylin, oxytocin, luteinizing hormone, (D-Tryp6)-LHRH, nafarelin acetate, leuprolide acetate, follicle stimulating hormone, glucagon, prostaglandins, steroids, estradiols, dexamethazone, testosterone, and other factors acting on the genital organs and their derivatives, analogs and congeners. As analogs of said LH-RH, such known substances as those described in U.S. Pat. Nos. 4,008,209, 4,086,219, 4,124,577, 4,317,815 and 5,110,904 can be mentioned.

Exemplary hematopoietic or thrombopoietic factors include, among others, erythropoietin, granulocyte colony stimulating factor (G-CSF), granulocyte-macrophage stimulating factor (GM-CSF) and macrophage colony stimulating factor (M-CSF), leukocyte proliferation factor preparation (Leucoprol, Morinaga Milk), thrombopoietin, platelet proliferation stimulating factor, megakaryocyte proliferation (stimulating) factor, and factor VIII.

Exemplary therapeutic factors acting on bone and skeleton and agents for treating osteoporosis include calcium, alendronate, bone GLa peptide, parathyroid hormone and its active fragments (osteostatin, Endocrinology 129, 324, 1991), histone H4-related bone formation and proliferation peptide (OGP, The EMBO Journal 11, 1867, 1992) and their muteins, derivatives and analogs thereof.

Exemplary enzymes and enzyme cofactors include: pancrease, L-asparaginase, hyaluronidase, chymotrypsin, trypsin, tPA, streptokinase, urokinase, pancreatin, collagenase, trypsinogen, chymotrypsinogen, plasminogen, streptokinase, adenyl cyclase, and superoxide dismutase (SOD).

Exemplary vaccines include Hepatitis B, MMR (measles, mumps, and rubella), and Polio vaccines.

Exemplary growth factors include nerve growth factors (NGF, NGF-2/NT-3), epidermal growth factor (EGF), fibroblast growth factor (FGF), insulin-like growth factor (IGF), transforming growth factor (TGF), platelet-derived cell growth factor (PDGF), hepatocyte growth factor (HGF) and so on.

Exemplary factors acting on the cardiovascular system include factors which control blood pressure, arteriosclerosis, etc., such as endothelins, endothelin inhibitors, endothelin antagonists described in EP 436189, 457195, 496452 and 528312, JP [Laid Open] No. H-3-94692/1991 and 130299/1991, endothelin producing enzyme inhibitors vasopressin, renin, angiotensin I, angiotensin II, angiotensin III, angiotensin I inhibitor, angiotensin II receptor antagonist, atrial naturiuretic peptide (ANP), antiarrythmic peptide and so on.

Exemplary factors acting on the central and peripheral nervous systems include opioid peptides (e.g. enkephalins, endorphins), neurotropic factor (NTF), calcitonin gene-related peptide (CGRP), thyroid hormone releasing hormone (TRH), salts and derivatives of TRH [JP [Laid Open] No. 50-121273/1975 (U.S. Pat. No. 3,959,247), JP [Laid Open] No. 52-116465/1977 (U.S. Pat. No. 4,100,152)], neurotensin and so on.

Exemplary factors acting on the gastrointestinal system include secretin and gastrin.

Exemplary factors acting on humoral electrolytes and hemal substances include factors which control hemagglutination, plasma cholesterol level or metal ion concentrations, such as calcitonin, apoprotein E and hirudin. Laminin and intercellular adhesion molecule 1 (ICAM 1) represent exemplary cell adhesion factors.

Exemplary factors acting on the kidney and urinary tract include substances which regulate the function of the kidney, such as brain-derived natriuretic peptide (BNP), urotensin and so on.

Exemplary factors which act on the sense organs include factors which control the sensitivity of the various organs, such as substance P.

Exemplary chemotherapeutic agents, such as paclitaxel, mytomycin C, BCNU, and doxorubicin.

Exemplary factors acting on the immune system include factors which control inflammation and malignant neoplasms and factors which attack infective microorganisms, such as chemotactic peptides and bradykinins.

Exemplary factors acting on the respiratory system include factors associated with asthmatic responses, e.g., albuterol, fluticazone, ipratropium bromide, beclamethasone, and other beta-agonists and steroids.

Also included are naturally occurring, chemically synthesized or recombinant peptides or proteins which may act as antigens, such as cedar pollen and ragweed pollen. These factors are administered, either independently, coupled to haptens, or together with an adjuvant, in the formulations according to the present invention.

Particularly preferred therapeutic molecules include, but are not limited to, betaxolol™, diclofenac™, doxorubicin, rifampin™, leuprolide acetate, luteinizing hormone releasing hormone (LHRH), (D-Tryp6)-LHRH, nafarelin acetate, insulin, sodium insulin, zinc insulin, proinsulin, C-peptide insulin, a mixture of insulin and C-peptide of insulin, hybrid insulin cocrystals, protamine, lysozyme, alpha-lactalbumin, basic fibroblast growth factor (bFGF), beta-lactoglobulin, trypsin, carbonic anhydrase, ovalbumin, bovine serum albumin (BSA), human serum albumin (HSA), phosphorylase b, alkaline phosphatase, beta -galactosidase, IgG, fibrinogen, nucleic acid molecules (e.g., complexed with poly-L-lysine), IgM, DNAase, desmopressin acetate™, growth hormone releasing factor (GHRF), somatostatin, antide, Factor VIII, G-CSF/GM-CSF, human growth hormone (hGH), beta interferon, antithrombin III, alpha interferon, alpha interferon 2b, parathyroid hormone, calcitonin, alpha1-antitrypsin, and formoterol.

Insulin or an insulin analog is a particularly preferred protein for use in accordance with the methods and compositions of the invention. As used herein, "insulin", refers to mammalian insulin, such as bovine, porcine or human insulin, whose sequences and structures are known in the art. The amino acid sequence and spatial structure of human insulin are well-known. Human insulin is comprised of a twenty-one amino acid A-chain and a thirty amino acid B-chain which are cross-linked by disulfide bonds. A properly cross-linked human insulin contains three disulfide bridges: one between position 7 of the A-chain and position 7 of the B-chain, a second between position 20 of the A-chain and position 19 of the B-chain, and a third between positions 6 and 11 of the A-chain.

The term "insulin analog" means proteins that have an A-chain and a B-chain that have substantially the same amino acid sequences as the A-chain and B-chain of human insulin, respectively, but differ from the A-chain and B-chain of human insulin by having one or more amino acid deletions, one or more amino acid replacements, and/or one or more amino acid additions that do not destroy the insulin activity of the insulin analog.

One type of insulin analog, "monomeric insulin analog" is well known in the art. These reportedly are fast-acting analogs of human insulin, including, for example, monomeric insulin analogs wherein: a) the amino acid residue at position B28 is substituted with Asp, Lys, Leu, Val, or Ala, and the amino acid residue at position B29 is Lys or Pro; b) the amino acid residues at positions B28, B29, and B30 are deleted; or c) the amino acid residue at position B27 is deleted. A preferred monomeric insulin analog is $Asp^{B28}$. An even more preferred monomeric insulin analog is $Lys^{B28}Pro^{B29}$.

Monomeric insulin analogs are disclosed in Chance, et al., U.S. Patent No. 5,514,646; Chance, et al., U.S. Patent Application Serial No. 08/255,297; Brems, et al., Protein Engineering, 5:527-533 (1992); Brange, et al., EPO Publication No. 214,826 (published March 18, 1987); and Brange, et al., Current Opinion in Structural Biology, 1:934-940 (1991). These disclosures are expressly incorporated herein by reference for describing monomeric insulin analogs.

Insulin analogs may also have replacements of the amidated amino acids with acidic forms. For example, Asn may be replaced with Asp or Glu. Likewise, Gln may be replaced with Asp or Glu. In particular, Asn(A18), Asn(A21), or Asp(B3), or any combination of those residues, may be replaced by Asp or Glu. Also, Gln(A15) or Gln(B4), or both, may be replaced by either Asp or Glu.

The preferred microspheres of the invention are produced by mixing proteins in an aqueous mixture with a water soluble polymer or mixture of polymers, thereafter contacting the solution with an energy source, preferably heat, under conditions sufficient to form the microspheres. The solution is preferably an aqueous solution. Either the protein solution is added to the polymer, or the polymer solution is added to the protein solution. Although not wishing to be bound to any particular theory or mechanism, it is believed that the polymer causes removal of water from, or dehydration of, the protein. This process is also referred to by those skilled in the art as volume exclusion or molecular crowding.

The protein and polymer solution is then exposed to an energy source, such as heat, radiation, including microwave radiation, or ionization, alone or in combination with sonication, vortexing, mixing or stirring, for a predetermined length of time to form and stabilize the microspheres. The resulting microspheres are then separated from any unincorporated components present in the solution by physical separation methods well known to those skilled in the art, and may then be washed or exposed to other drug-containing solutions for binding of additional drugs to the microspheres.

The length of incubation time is dependent upon the respective concentrations of polymer and protein and the level of energy of the energy source. Microsphere stabilization can begin to occur immediately upon exposure to the energy source. Preferably, the protein and polymer mixture is heated at a temperature greater than room temperature for between approximately 1 minute and 24 hours. Most preferably, the polymer and proteins are heated for 30 minutes or less at a temperature between approximately 37°C and 99°C.

An organic or inorganic natural or synthetic pharmaceutical compound or drug may be incorporated into the microspheres by binding the drug to a protein, and then forming the microspheres from the protein-drug complex or conjugate. It will be understood by those skilled in the art that a compound incapable of having a tertiary structure can be formed into a microsphere by incorporation or binding of the compound into a carrier molecule that has a tertiary structure. It will also be understood that the term "protein" includes a plurality of proteins and includes combinations of different proteins such as a combination of a pharmaceutical compound and an affinity molecule for targeting the pharmaceutical compound to a tissue, organ or tumor requiring treatment. It will be further understood that an affinity molecule can be either the receptor portion or the ligand portion of a receptor-ligand interaction. Examples of ligands that interact with other biomolecules include viruses, bacteria, polysaccharides, or toxins that may act as antigens to generate an immune response when administered to an animal and cause the production of antibodies.

Suitable compounds that can be attached to proteins or proteins that can be used in accordance with the methods and compositions of the invention include, but are not limited to, betaxolol™, diclofenac™, doxorubicin, rifampin™, leuprolide acetate, luteinizing hormone releasing hormone (LHRH), (D-Tryp6)-LHRH, nafarelin acetate, insulin, sodium insulin, zinc insulin, protamine, lysozyme, alpha-lactalbumin, basic fibroblast growth factor (bFGF), beta-lactoglobulin, trypsin, calcitonin, parathyroid hormone, carbonic anhydrase, ovalbumin, bovine serum albumin (BSA), human serum albumin (HSA), phosphorylase b, alkaline phosphatase, beta -galactosidase, IgG, fibrinogen, poly-L-lysine, IgM, DNA, desmopressin acetate, growth hormone releasing factor (GHRF), somatostatin, antide, Factor VIII, G-CSF/GM-CSF, human growth hormone (hGH), beta interferon, antithrombin III, alpha interferon, alpha interferon 2b. See also the above-list of protein carrier and protein therapeutic agents.

The incubation conditions are optimized to maximize incorporation of the protein into the microspheres and retention of activity by adjusting the pH, temperature, ionic strength, concentration of protein and/or polymer, or duration of reaction or incubation.

As mentioned above, a small molecule or compound, such as a peptide or pharmaceutical compound, can be formed into a microsphere by incorporation or binding of the compound into a protein which has a tertiary structure. This may be achieved in several ways. For example, microspheres may be formed as described herein using a protein having a tertiary structure, and then the small molecule or compound is bound inside and/or on the surface of the microsphere. Alternatively, the small molecule or compound is bound to the protein having a tertiary structure using hydrophobic or ionic interactions, and then microspheres are formed from the protein-small molecule complex using the method described herein. This category of embodiment includes the complexation of a nucleic acid molecule (e.g., DNA or an anti-sense molecule) with a polyaminoacid (e.g., a poly-lysine, polyarginine). A third way to make microspheres from small molecules is to prepare microspheres using a protein having a tertiary structure in such a way that the microsphere has a net charge and then add a small molecule or compound having an opposite net charge so that the small molecule is physically attracted to and remains attached to the microsphere, but can be released over time under the appropriate conditions. Alternatively, different types of covalent or non-covalent interactions such as hydrophobic or affinity interactions may be used to allow attachment and subsequent release of small molecules.

When preparing microspheres containing a protein, a protein stabilizer such as glycerol, fatty acids, sugars such as sucrose, ions such as zinc, sodium chloride, calcium chloride, or any other protein stabilizers known to those skilled in the art may be added prior to the addition of the polymers during microsphere formation to minimize protein denaturation.

Molecules, distinct from the proteins of which the microspheres are composed, may be attached to the outer surface of the microspheres by methods known to those skilled in the art to "coat" or "decorate" the microspheres. The microspheres can have a molecule attached to their outer surface. These molecules are attached for purposes such as to facilitate targeting, enhance receptor mediation, and provide escape from endocytosis or destruction, and to alter their release kinetics. For example, biomolecules such as phospholipids may be attached to the surface of the microsphere to prevent degradation in circulation and/or to promote or inhibit interaction with biological membranes, endocytosis by endosomes; receptors, antibodies or hormones may be attached to the surface to promote or facilitate targeting of the microsphere to the desired organ, tissue or cells of the body; and polysaccharides, such as glucans, or other polymers, such as polyvinyl pyrrolidone and PEG, may be attached to the outer surface of the microsphere to enhance or to avoid uptake by macrophages.

In addition, one or more cleavable, erodable or soluble molecules may be attached to the outer surface of or within the microspheres. The cleavable molecules are designed so that the microspheres are first targeted to a predetermined site under appropriate biological conditions and then, upon exposure to a change in the biological conditions, such as a pH change, the molecules are cleaved causing release of the microsphere from the target site. In this way, microspheres are attached to or taken up by cells due to the presence of the molecules attached to the surface of the microspheres. When the molecule is cleaved, the microspheres remain in the desired location, such as within the cytoplasm or nucleus of a cell, and are free to release the proteins of which the microspheres are composed. This is particularly useful for drug delivery, wherein the microspheres contain a drug that is targeted to a specific site requiring treatment, and the drug can be slowly released at that site.

With respect to non-pulmonary applications, the microspheres may be coated with one or more stabilizing substances, which may be particularly useful for long term depoting with parenteral administration or for oral delivery by allowing passage of the microspheres through the stomach or gut without dissolution. For example, microspheres intended for oral delivery may be stabilized with a coating of a substance such as mucin, a secretion containing mucopolysaccharides produced by the goblet cells of the intestine, the submaxillary glands, and other mucous glandular cells.

Additionally, the microspheres can be covalently or non-covalently coated with compounds such as fatty acids, lipids, or polymers. The coating may be applied to the microspheres by immersion in the solubilized coating substance, spraying the microspheres with the substance or other methods well known to those skilled in the art.

The pulmonary compositions of the invention are prepared by contacting the microparticles (e.g., microspheres) with a propellant (e.g., a hydrofluoroalkane propellant) to form a suspension and, thereafter, agitating the suspension for a time sufficient to suspend the microparticles in the propellant. Preferably, the compositions of the invention are characterized in that the microspheres remain in suspension a minimum of 10 seconds to 10 minutes, preferably, at least 1 to 10 hours, and more preferably, at least 1 to 7 days following agitation.

In preferred embodiments, the pulmonary compositions have a density ratio of $\rho_{microparticle}$ to $\rho_{propellant}$ in the range of .05 to 30 and, more preferably, in the range of 0.5 to 3.0. The density ratio is described in more detail below. The embodiments optionally contain a surfactant. Preferably, the propellant is an HFA (hydrofluoroalkane) propellant such as HFA P134a, HFA P227, or a blend of these or other propellants.

Although it is preferred not to include a surfactant in the pulmonary formulations disclosed herein, a surfactant can be added if desired. As used herein, a surfactant is a term of art that refers to an agent which preferentially adsorbs to an interface between two immiscible phases, such as the interface between water and an organic polymer solution, a water/air interface, an organic solvent/air interface, or microparticle/propellant interface.

Surfactants generally possess a hydrophilic moiety and a lipophilic moiety, such that, upon absorbing to microspheres, they tend to present moieties to the external environment that do not attract similarly-coated particles, thus reducing particle agglomeration. Surfactants may also promote absorption of a therapeutic or diagnostic agent and increase bioavailability of the agent.

Synthetic or naturally occurring surfactants known in the art, include phosphoglycerides. Exemplary phosphoglycerides include phosphatidylcholines, such as the naturally occurring surfactant, L-$\alpha$ phosphatidylcholine dipalmitoyl ("DPPC"). The use of surfactants endogenous to the lung may avoid the need for the use of non-physiologic surfactants. Other exemplary surfactants include diphosphatidyl glycerol (DPPG); sodium dodecyl sulfate (SDS), polyethylene glycol (PEG) and its derivatives; polyvinylpyrrolidone (PVP) and its derivatives; polyoxyethylene-9-lauryl ether; a surface active fatty acid, such as palmitic acid or oleic acid; sorbitan trioleate (Span 85); glycocholate; surfactin; poloxamers; sorbitan fatty acid esters such as sorbitan trioleate; tyloxapol and a phospholipid; and alkylated sugars such as octyl glucoside.

It is to be understood that any of the preferred embodiments with respect to the first aspect of the invention are applicable to other aspects of the invention; however, for the sake of conciseness, the various preferred embodiments are not repeated for each aspect of the invention. The invention provides compositions and methods in which any one or more limitations which represent a preferred embodiment can be used in combination with any other limitation in each aspect of the invention.

According to a second aspect of the invention, a composition comprising: a plurality of microparticles (e.g., microspheres), said microparticles containing a protein; and a propellant (e.g., a hydrofluoroalkane (HFA) propellant), is provided; however in this aspect, the composition does not contain a surfactant. Preferably, the composition has a fine particle fraction in the range of 25% to 100%.

According to a third aspect of the invention, a method for preparing a pulmonary preparation is provided. The method involves: 1) selecting a propellant, such as a hydrofluoroalkane propellant having a known density, $\rho_{propellant}$ (e.g., $\rho_{hydrofluoroalkane}$); 2) selecting a microparticle (e. g., microsphere) having a microparticle density $\rho_{microparticle}$ (e.g., $\rho_{microsphere}$) such that the ratio of $\rho_{microparticle}$ to $\rho_{propellant}$ is in the range of .05 to 30 and, more preferably, in the range of 0.5 to 3.0; and 3) contacting a plurality of the microspheres with the propellant to form the pulmonary preparation. Preferably, the propellant is an HFA propellant such as HFA P134a, HFA P227, or a blend of these propellants. In these and other embodiments, the composition preferably does not include a surfactant.

As used herein, the term "$\rho_{propellant}$" refers to the density of the propellant. In general, such densities are published for these commercially available agents. Similarly, the phrase, "microsphere density, $\rho_{microsphere}$", refers to the density of the microspheres. Microsphere density values are published for commercially available microspheres and/or can be determined in accordance with standard methods known to those of ordinary skill in the art. Thus, the density of the microspheres is selected as discussed above to have a ratio which falls within the above-prescribed range. Preferably, the hydrofluoroalkane propellant is an HFA propellant such as HFA P134a, HFA P227, or a blend of these propellants. In certain preferred embodiments, the composition does not include a surfactant.

According to a fourth aspect of the invention, a method of administering a protein to the pulmonary system of a subject is provided. The method involves administering to the respiratory tract of a subject in need of treatment, an effective amount of a composition of the invention to treat the condition. In certain embodiments, the composition of the first aspect of the invention is administered; in yet other embodiments, the composition of the second aspect of the invention is provided.

In a particularly preferred embodiment, the microparticles (e.g., microspheres) contain insulin. The following description provides general methods for making and using insulin microspheres; however, it is to be understood that these methods are illustrative only and that other proteins can be used in place of insulin and that other microparticles can be used in place of the microspheres to prepare the compositions of the invention. Specific procedures for making insulin microspheres are provided in the Examples.

In a preferred embodiment, insulin is administered by inhalation in a dose effective manner to increase circulating insulin protein levels and/or to lower circulating glucose levels. Such administration can be effective for treating disorders such as diabetes or hyperglycemia. Achieving effective doses of insulin requires administration of an inhaled dose of more than about 0.5 $\mu$g/kg to about 500 $\mu$g/kg insulin, preferably about 3 $\mu$g/kg to about 50 $\mu$g/kg, and most preferably about 7 $\mu$g/kg to about 25 $\mu$g/kg. A therapeutically effective amount can be determined by a knowledgeable practitioner, who will take into account factors including insulin level, blood glucose levels, the physical condition of the patient, the patient's pulmonary status, or the like.

According to the invention, insulin is delivered by inhalation to achieve either or both of rapid absorption or slow absorption by sustained release of this protein. Administration by inhalation can result in pharmacokinetics comparable or superior to subcutaneous administration of insulin. Inhalation of insulin leads to a rapid rise in the level of circulating insulin followed by a rapid fall in blood glucose levels. Different inhalation devices typically provide similar pharmacokinetics when similar particle sizes and similar levels of lung deposition are compared.

According to the invention, insulin can be delivered by any of a variety of inhalation devices known in the art for administration of a therapeutic agent by inhalation. These devices include metered dose inhalers, nebulizers, dry powder generators, sprayers, and the like. There are several desirable features of an inhalation device for administering insulin. For example, delivery by the inhalation device is advantageously reliable, reproducible, and accurate. The inhalation device should deliver small microspheres, e.g. less than about 10 $\mu$m, preferably about 0.2-5 $\mu$m, for good

respirability. Some specific examples of commercially available inhalation devices suitable for the practice of this invention are Turbuhaler™ (Astra, Wilmington, DE), Rotahaler® (Glaxo, Research Triangle Park, NC), Diskus® (Glaxo, Research Triangle Park, NC), Spiros™ inhaler (Dura, San Diego, CA), devices marketed by Inhale Therapeutics (San Carlos, CA), AERx™ (Aradigm, Hayward, CA), the Ultravent® nebulizer (Mallinckrodt, Hazelwood, MO), the Acorn II® nebulizer (Marquest Medical Products, Totowa, NJ), the Ventolin® metered dose inhaler (Glaxo, Research Triangle Park, NC), the Spinhalero® powder inhaler (Aventis, Bridgewater, NJ), and metered dose inhalers supplied by Bespak (London, UK); 3M (Minneapolis, MN); Valois (France), or the like.

The insulin microsphere in the formulation delivered by the inhalation device is critical with respect to the ability of the protein to make it into the lungs, and preferably into the lower airways or alveoli for systemic administration. Preferably, the insulin microspheres are formulated so that at least about 10% to 40% of the insulin delivered is deposited in the lung, preferably about 40% to about 50%, or more, and, more preferably, 70% to 80%, or more. It is known that the maximum efficiency of pulmonary deposition for mouth breathing humans is obtained with particles having aerodynamic diameters of about 0.1 $\mu$m to about 10 $\mu$m. When particle sizes are above about 5 $\mu$m, pulmonary deposition decreases substantially. Thus, microspheres of insulin delivered by inhalation have a particle size preferably less than about 10 $\mu$m, more preferably in the range of about 0.1 $\mu$m to about 5 $\mu$m, and most preferably in the range of about 0.1 $\mu$m to about 3 $\mu$m. The formulation of insulin microspheres is selected to yield the desired particle size in the chosen inhalation device.

Advantageously for administration, insulin is prepared in a microsphere with a size of less than about 10 $\mu$m, preferably about 0.1 to about 5 $\mu$m, and most preferably about 0.1 $\mu$m to about 3 $\mu$m. The preferred microsphere size is effective for delivery to the alveoli of the patient's lung. Preferably, the formulation is largely composed of microspheres produced so that a majority of the particles have a size in the desired range. Advantageously, at least about 90% of the formulation is made of particles having a diameter less than about 10$\mu$m. Such formulations can be achieved using the methods of the invention and those previously disclosed in U.S. Patent Nos. 6,090,925; 5,981,719; 5,578,709; pending, commonly assigned U.S. Patent Application No. 09/420,361, filed October 18, 1999; and U.S. Patent Application No. 60/244,098, filed October 27, 2000; or by selecting the preferred size distribution from a larger distribution of microparticles (e.g., microspheres).

Formulations of insulin for administration by inhalation typically include the insulin microspheres of the invention and, optionally, a bulking agent, surfactant, carrier, excipient, another additive, or the like. Additives can be included in the formulation of insulin microspheres, for example, to dilute the microspheres as required for delivery by inhalation, to facilitate processing of the formulation, to provide advantageous properties to the formulation, to facilitate dispersion of the formulation from the inhalation device, to stabilize the formulation (e.g., antioxidants or buffers), to provide taste to the formulation, or the like. The insulin microspheres can be mixed with an additive at a molecular level or the solid formulation can include insulin microspheres mixed with or coated on particles of the additive. Typical additives include mono-, di-, and polysaccharides; sugar alcohols and other polyols, such as, for example, lactose, glucose, raffinose, melezitose, lactitol, maltitol, trehalose, sucrose, mannitol, starch, or combinations thereof; surfactants, such as sorbitols, diphosphatidyl choline, or lecithin; or the like. Typically an additive, such as a bulking agent, is present in an amount effective for a purpose described above, often at about 50% to about 90% by weight of the formulation. Additional agents known in the art for formulation of a protein can be included in the formulation.

Administration of a formulation of insulin microspheres by inhalation is a preferred method for treating diabetes.

A spray including insulin microspheres can be produced by forcing a suspension of insulin microspheres suspended in a propellant or other liquid suspending agents through a nozzle under pressure. The nozzle size and configuration, the applied pressure, and the liquid feed rate can be chosen to achieve the desired output and droplet size using any inhalation device known to those of skill in the art. An electrospray or piezoelectric spray can be produced, for example, by an electric field in connection with a capillary or nozzle feed. Advantageously, insulin microspheres delivered by a sprayer have a particle size less than about 10 $\mu$m, preferably in the range of about 0.1 $\mu$m to about 5 $\mu$m, and most preferably about 0.1 $\mu$m to about 3 $\mu$m.

Formulations of insulin microspheres suitable for use with a nebulizer typically include an aqueous suspension of the microspheres at a concentration of about 1 mg to about 20 mg of insulin per ml of suspension. The formulation can include agents such as an excipient, a buffer, an isotonicity agent, a preservative, a surfactant, a polymer (e.g., polyethylene glycol), and, a metal ion such as zinc or calcium. The formulation can also include an excipient or agent for stabilization of the insulin, such as a buffer, a reducing agent, a bulk protein, or a carbohydrate. Bulk proteins useful in formulating insulin include albumin, protamine, or the like. Typical carbohydrates useful in formulating insulin include sucrose, mannitol, lactose, trehalose, glucose, or the like. In general, the insulin microsphere formulations do not contain a surfactant because the insulin microspheres do not have a tendency to aggregate.

Insulin microspheres can be administered by a nebulizer, such as a jet nebulizer or an ultrasonic nebulizer. Typically, in a j et nebulizer, a compressed air source is used to create a high-velocity air jet through an orifice. As the gas expands beyond the nozzle, a low-pressure region is created, which draws a suspension of insulin microspheres through a capillary tube connected to a liquid reservoir. The liquid stream from the capillary tube is sheared into unstable filaments and droplets as it exits the tube, creating the aerosol. A range of configurations, flow rates, and baffle types can be employed to achieve the desired performance characteristics from a given jet nebulizer. In an ultrasonic nebulizer, high-frequency electrical energy is used to create vibrational, mechanical energy, typically employing a piezoelectric transducer. This energy is transmitted to the formulation of insulin microspheres either directly or through a coupling fluid, creating an aerosol including the insulin microspheres. Advantageously, insulin microspheres delivered by a nebulizer have a particle size less than about 10 μm, preferably in the range of about 0.1 μm to about 5 μm, and most preferably about 0.1 μm to about 3 μm.

Formulations of insulin microspheres suitable for use with a nebulizer, either jet or ultrasonic, typically include insulin microspheres in a suspension at a concentration of about 1 mg to about 20 mg of insulin per ml of suspension. The formulation can include additional agents such as those mentioned above (e.g., excipients, buffers, and so forth).

In a metered dose inhaler (MDI), a propellant, insulin microspheres, and any excipients or other additives are contained in a canister as a mixture including a liquefied compressed gas. Actuation of the metering valve releases the mixture as an aerosol, preferably containing microspheres in the size range of less than about 10 μm, preferably about 0.1 μm to about 5 μm, and most preferably about 0.1 μm to about 3 μm. The desired microsphere size can be obtained by employing a formulation of insulin produced by the methods disclosed herein. Preferred metered dose inhalers include those manufactured by Bespak, Valois, 3M or Glaxo and employing a propellant.

Formulations of insulin microspheres for use with a metered-dose inhaler device will generally include the microspheres as a suspension in a non-aqueous medium, for example, suspended in a propellant. In general, a surfactant is not needed because the insulin microspheres disclosed herein have a consistent size and do not have a tendency to aggregate. The propellant may be any conventional material employed for this purpose, such as a chlorofluorocarbon, including trichlorofluoromethane, dichlorodifluoromethane, dichlorotetrafluoroethanol; and a hydrofluoroalkane, including HFA P134a (1,1,1,2-tetrafluoroethane), HFAP227 (1,1,1,2,3,3,3-heptafluoropropane-227); or any other propellant that is useful for practicing the invention. Preferably the propellant is a hydrofluoroalkane. Additional agents known in the art for formulation of a protein such as insulin can also be included in the formulation.

One of ordinary skill in the art will recognize that the methods of the current invention may be achieved by pulmonary administration of insulin microspheres via devices not described herein.

According to a fifth aspect of the invention, a method of manufacture is provided. The method involves dispersing one or more therapeutic doses into a pulmonary delivery device, said therapeutic doses containing a therapeutically effective amount of a composition of the invention. As used herein, a "therapeutically effective amount" refers to that amount of active agent necessary to delay the onset of, inhibit the progression of, or alleviate the particular condition being treated. Generally, a therapeutically effective amount will vary with the subject's age, condition, and sex, as well as the nature and extent of the disease in the subject, all of which can be determined by one of ordinary skill in the art. The dosage may be adjusted by the individual physician or veterinarian, particularly in the event of any complication. A therapeutically effective amount of active agent typically varies from 1 pg/kg to about 1000 mg/kg, preferably from about 1 μg/kg to about 200 mg/kg, and most preferably from about 0.1 mg/kg to about 20 mg/kg, in one or more dose administrations daily, for one or more days, weekly, monthly, every two or three months, and so forth.

The microspheres may be administered alone or in combination with other drug therapies as part of a pharmaceutical composition. Such a pharmaceutical composition may include the microspheres in combination with any standard physiologically and/or pharmaceutically acceptable carriers which are known in the art. The compositions may be sterile and contain a therapeutically effective amount of the microsphere in a unit of weight or volume suitable for administration to a patient. The term "pharmaceutically-acceptable carrier" as used herein means one or more compatible solid or liquid filler, diluents or encapsulating substances which are suitable for administration into a human or other animal. The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction which would substantially impair the desired pharmaceutical efficacy. Pharmaceutically acceptable further means a non-toxic material that is compatible with a biological system such as a cell, cell culture, tissue, or organism. The characteristics of the carrier will depend on the route of administration. Physiologically and pharmaceutically acceptable carriers include diluents, fillers, salts, buffers, stabilizers, desiccants, bulking agents, propellants, acidifying agents, coating agents, solubilizers, and other materials which are well known in the art. Carrier formulations suitable for oral, subcutaneous, intravenous, intramuscular, etc. administrations can be found in Remington's Pharmaceutical Sciences, Mack Publishing Co., Easton, PA.

Thus, the invention provides various pharmaceutical compositions of matter and method for producing same. In general, the compositions include a container containing one or more doses of microspheres containing an active agent for treating a condition that is treatable by the release of an active agent from the microspheres. The number of microspheres in the single dose is dependent upon the amount of active agent present in each microsphere and the period of time over which release is desired. Preferably, the single dose is selected to achieve a duration of release of the active agent over a period of 0.1 hours to 96 hours with the desired release profile.

According to a sixth aspect of the invention, a further method of manufacture is provided. The method involves dispersing one or more therapeutic doses into a package for use with a pulmonary delivery device, said therapeutic doses containing a therapeutically effective amount of a composition of the invention.

The package preferably contains between one or two and five-hundred therapeutic doses of the microspheres for treating a condition that is treatable by the release of the active agent *in vivo.* The number of microspheres present in the single dose is dependent on the type and activity of the active agent. Preferably, a single dose is selected to achieve release over a period of time which has been optimized for treating the particular medical condition.

According to a seventh and related aspect of the invention, a package including a container containing one or more therapeutic doses of an above-noted composition of the invention is provided. The package preferably provides instructions for using the container to deliver its contents to a pulmonary delivery device and, optionally, additional instructions for using the inhaler device according to manufacturer's instructions.

Although pulmonary delivery of the microsphere formulations is a particularly preferred aspect of the invention, it is to be understood that the microspheres disclosed herein can be delivered to a subj ect in accordance with methods known in the art for delivering microspheres to a subject, and particularly a human patient in need of medical treatment. Suitable delivery routes include parenteral, such as intramuscular (i.m.), intravascular (i.v.) and subcutaneous (s.c.), and non-parenteral, such as oral, buccal, intrathecal, nasal, pulmonary, transdermal, transmucosal, and the like. Delivery devices include syringes, both needleless and needle containing, as well as inhalation devices. Thus, although pulmonary delivery is preferred, the microspheres of this invention can be delivered orally, intranasally, intravenously intramuscularly, subcutaneously, and by other delivery methods suitable for the delivery of therapeutic molecules.

The microspheres may be administered alone or in combination with other drug therapies as part of a pharmaceutical composition. Such a pharmaceutical composition may include the microspheres in combination with any standard physiologically and/or pharmaceutically acceptable carriers which are known in the art. The compositions may be sterile and contain a therapeutically effective amount of the microspheres in a unit of weight or volume suitable for administration to a patient.

The pharmaceutical compositions may conveniently be presented in unit dosage form and may be prepared by any of the methods well-known in the art of pharmacy. All methods may or may not include the step of bringing the microspheres into association with a carrier which constitutes one or more accessory ingredients. The compositions may be prepared by uniformly and intimately bringing the microspheres into association with a liquid carrier, a finely divided solid carrier, or both, and then, if necessary, shaping the product.

Preparations for parenteral administration include sterile aqueous or non-aqueous solutions, suspensions, and emulsions. Additional examples of solvents or suspending agents include propylene glycol, ethanol, polyethylene glycol, vegetable oils such as olive oil, and injectable organic esters such as ethyl oleate. Aqueous carriers include water, salts and buffer solutions such as saline and buffered media, alcoholic/aqueous solutions and emulsions or suspensions. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. In general, the microspheres can be administered to the subject (any animal recipient) using the same modes of administration that currently are used for microparticle (e.g., microsphere) therapy in humans.

The microspheres are useful as therapeutic agents and may enable the use of alternative routes of administration when the microspheres include a therapeutic drug and are administered to a patient for release or targeted delivery of the drug to the site requiring therapy. The microspheres are also useful as therapeutic or prophylactic agents when the microspheres include a protein that is itself a therapeutic or prophylactic agent, such as an enzyme or immunoglobulin. The release of such therapeutic agents is particularly useful for therapeutic proteins or peptides having short half-lives that must be administered by injection.

The microspheres are useful for therapy or prophylaxis when the protein is a therapeutic agent or a pharmaceutical compound that is delivered to a patient and released from the microspheres over time. These microspheres may be particularly useful for slow release of drugs with short biological half-lives, such as proteins or peptides. If the pharmaceutical compound cannot be formed into a particle, then it is complexed to a carrier, such as albumin, and the carrier-pharmaceutical compound complex is formed into a microsphere. The microsphere can either provide for the release of the agent throughout the body or the microsphere can include an affinity molecule specific for a target tissue, or tumor, and be injected into a patient for targeted release of the therapeutic agent, such as an antitumor, antiviral, antibacterial, antiparasitic, or antiarthritic agent, cytokine, hormone, or insulin directly to the site requiring therapy.

In addition to the above-described therapeutic applications, the microspheres described herein are useful as solid phase particles in an assay, such as an enzyme-linked immunosorbant assay, dot-blot, or Western blot, for the detection of a particular target such as a cell, biomolecule or drug in a biological sample. The microspheres designed for this use are composed of affinity molecules specific for the target molecule. For example, the protein is an immunoglobulin or cell receptor and is bound to a test tube or microtiter plate.

For detection or quantitation of a target molecule of interest, a sample is combined with a solution containing the microspheres, the proteins on the microspheres are reacted with the target molecule, the microspheres are separated from any non-bound components of the sample, and microspheres containing bound molecules are detected by conventional methods. Fluorescently stained microspheres are particularly well suited for flow cytometry analysis in accordance with methods well known to those skilled in the art.

The microspheres described herein are useful as visual probes or markers of pathology in a histological sample. The proteins of the microspheres designed for this use are specific for biomolecules expressed during a particular pathologic condition and are labeled with a detectable label. For example, the protein is an immunoglobulin or cell receptor specific for an abnormal cell, such as a rapidly proliferating cell, or pathological organism, for example, a virus.

For detection of a pathogenic condition, a histological sample is combined with a solution containing the microspheres, the labeled proteins on the microspheres are reacted with the target molecule of interest, and bound microspheres are detected by detecting the label in accordance with methods well known to those skilled in the art.

The microspheres described herein are useful as imaging agents for in vivo localization of a particular molecule, cell type or pathologic condition in a manner similar to that described above with regard to the use of the microspheres for histopathology. The proteins on microspheres designed for this use are specific for molecules expressed by a particular cell or pathologic organism and are labeled with a detectable label. For example, the protein is an immunoglobulin specific for a tumor cell or pathological organism, such as a virus.

The microspheres are used to either detect a pathologic condition or to monitor the success of therapy, such as chemotherapy or surgery to ensure that the size of an abnormal tissue tumor has decreased or has been completely excised. For this use, a patient receives an administration of a microsphere formulation and, preferably, the labeled proteins on the microspheres are given a sufficient amount of time to localize to the affected organ or region of the body, the protein is reacted with a target molecule expressed by the cell or organism under investigation, and bound microspheres are detected by detecting the label by conventional imaging techniques well known to those skilled in the art, such as X-ray.

The microspheres are useful for therapy or prophylaxis when the protein is a therapeutic agent that is delivered to a patient. These microspheres are particularly useful for slow release of drugs with short biological half-lives, such as proteins or peptides. If the pharmaceutical compound cannot be formed into a particle, then it is complexed to a carrier, such as albumin, and the carrier-pharmaceutical compound complex is formed into a microsphere. The microsphere can either provide for the slow release of the agent throughout the body or the microsphere can include an affinity molecule specific for a target tissue, or tumor, and be injected into a patient for targeted slow release of the therapeutic agent, such as an antitumor, antiviral, antibacterial, antiparasitic, or antiarthritic agent, cytokine, hormone, or insulin directly to the site requiring therapy. The affinity molecule may be cleavable.

Microspheres composed of antigenic proteins or polysaccharide-protein conjugates capable of provoking an immune response are particularly suitable for use as vaccines.

The microspheres are useful as research tools for the purification of a biomolecule from a complex mixture, as a reagent for the detection or quantification of a biomolecule, or for the production of biomolecules, such as antibodies.

For example, microspheres composed of a protein, such as an immunoglobulin, are attached to a chromatography column and used in immunoaffinity chromatography to separate a ligand from a complex mixture. It will be understood by those skilled in the art that microspheres for use in high pressure liquid chromatography should be first attached to a non-compressible solid phase sphere or bead so that the column packing maintains its rigid structure under pressure.

Alternatively, microspheres including a labeled macromolecule or a mixture of labeled macromolecules specific for different cells or cell receptors are used to detect changes in the number of cells or cell surface receptors in response to a particular test condition using techniques such as flow cytometry.

The following Examples are illustrative of certain embodiments of the invention, and are intended to further describe the present invention, without limiting it thereby. Various modifications can be made to these embodiments without departing from the spirit or scope of the invention. It is to be understood that generic brands of reagents and equipment can be used in place of any of the specific brands identified herein. The Examples refer to, and include descriptions of the figures. It is to be understood that the figures in the Examples are not required for enablement of the claimed invention.

## Examples

Example 1

*Method for production of Insulin pulmonary microspheres in 1.5 mL microcentrifuge tube.*

One ml of 10 mg/ml insulin solution was prepared (this solution is prepared just prior to use). Per milliliter of solution, 10 mg of insulin (Zn) was mixed in 0.99 mL of degassed deionized water. The suspension would be cloudy.

10 microliters of 1 N HCl per mL of solution was added and mixed. The solution should clear with mixing. If the solution did not clear, smaller volumes of 1N HCl were added until the insulin was in solution. 0.8 ml of PEG/PVP (12.5%/12.5% PVP in 0.1 M Sodium acetate, pH 5.65) in 0.1 M Sodium acetate, pH 5.65 was added to 0.40 mL of insulin solution and mixed gently in a 1.5 mL polypropylene microcentrifuge tube. The solution turned cloudy.

The microcentrifuge tube was placed into the 90 °C water bath for 30 minutes. The microcentrifuge tube was removed from the water bath and cooled on bench at room temperature for 30 minutes. The microcentrifuge tube was centrifuged in a microcentrifuge for 10 minutes at 8000 RPM. The supernatant was decanted. Deionized water was added and the pellet was resuspended. The microcentrifuge tube was centrifuged in a microcentrifuge for 10 minutes at 6000 RPM. The supernatant was decanted. Deionized water was added, the pellet was resuspended and repeated. The microsphere pellet was resuspended in 5 mL of deionized water and the pellet was lyophilized. The resulting lyophilized spheres yielded 1 micron sized Insulin spheres by laser light scattering which assayed to be > 95% wt/wt insulin.

Example 2

*Method for Fabricating Insulin Pulmonary Microspheres via continuous flow through.*

Ten ml of 10 mg/ml insulin solution was prepared (this solution was prepared just prior to use). Per milliliter of solution, 100 mg of insulin (Zn) was mixed in 9.8 mL of degassed deionized water. The suspension would be cloudy. 100 microliters of 1 N HCl was added per mL of solution and mixed. The solution would clear with mixing. If the solution did not clear, smaller volumes of 1N HCl were added until the insulin was in solution. 20 ml of PEG/PVP (12.5%/12.5%) in 0.1 M Sodium acetate, pH 5.65 was added to 10 mL of insulin solution and mixed gently. The solution would turn cloudy.

Fabrication apparatus set-up: Eight feet of 1/8 inch o.d. (3/32 i.d.) polypropylene tubing was prepared. It was ensured that 4 feet were submerged in the water bath in a loop of about 6 inches diameter, with the inlet connected to the Rainin peristaltic pump and the outlet to an empty collection vessel. A three (3) foot cooling loop was allowed between the water bath and the collection vessel. The water bath was heated to 90 °C. Note: It is important to not allow any air to enter tubing after beginning to pump solutions through the tubing. Air bubbles can cause aggregation and clogging.

Microsphere production procedure: The Rainin peristaltic pump speed was set to a setting that is approximately a 1 mL/minute flow rate. Immediately prior to starting the run, about 10 mL of the diluted, degassed polymer solution (1 part deionized water to 2 parts PEG/PVP (12.5%/12.5%) was pumped in 0.1 M sodium acetate, pH 5.65) through the tubing to equilibrate the temperature of the cooling zone. The pump was stopped momentarily to avoid drawing a bubble into the tubing. The inlet side of the tubing was carefully transferred to the insulin/polymer raw material suspension. The collection vessel was switched to an empty container before the Insulin microspheres exited the tubing. The first Insulin microspheres exited the tubing much faster than expected from the actual flow rate of the pump due to laminar flow. A thin line of Insulin raw material suspension was observed running through the pre-filled polymer solution in the tube, that gradually widened to the inside diameter of the tubing. A small air bubble was allowed, then the polymer/buffer solution which has been diluted by one third (1:3) with deionized water to pump through tubing following the insulin-PEG/PVP solution. The collection vessel was removed for further processing after the microsphere collection was completed.

Microsphere washing procedure: The microsphere suspension was diluted with approximately an equal volume of deionized water in order to reduce the viscosity of the suspension. Using a 50 mL polypropylene centrifuge tube, the microsphere suspension was spun at 3500 x g for 15 minutes. The supernatant was carefully decanted from the pellet. The pellet in each tube was resuspended with deionized water equivalent to the original volume in the tube and then vortexed until all of the pellet has been completely resuspended. The suspension was centrifuged at 3000 x g for 15 minutes. The supernatant was carefully decanted from the pellet. The pellet in each tube was resuspended with deionized water equivalent to the original volume in the tube and then vortexed until all of the pellet has been completely resuspended. The suspension was centrifuged at 3000 x g for 15 minutes. The supernatant was carefully decanted from the pellet. The pellet in each tube was resuspended with deionized water equivalent to 2/3 the volume of the tube and then vortexed until all of the pellet has been completely resuspended. The suspension was homogenized with a homogenizer (e.g., IKA Homogenizer at speed setting 3 for 2 minutes) and centrifuged at 3000 x g for 15 minutes. The supernatant was carefully decanted from the pellet. The pellet in each tube was resuspended with a minimum volume of deionized water and then vortexed until all of the pellet has been completely resuspended. The suspension was homogenized with the IKA Homogenizer at speed setting 3 for 2 minutes or equivalent. The microspheres were transferred to an appropriate sterile vessel and diafiltration was performed to wash microspheres until all free polymer has been removed. The microsphere suspension was concentrated using the hollow fiber cartridge system prior to lyophilization. The microspheres were bulk lyophilized under sanitary conditions, and stored dry until ready for filling.

Example 3

*Method for production of Insulin pulmonary microspheres in 2 foot length (60 mL) glass chromatography column (general batch-wise process).*

The water jacketed chromatography column was preheated to 90 °C. A 10 mg/mL insulin solution was prepared in degassed, deionized water as described in Example 1. A 12.5% PEG (3350), 12.5% PVP (K12) in 0.1 M sodium acetate buffer was prepared. 20 mL of the insulin solution was mixed with 40 mL of the polymer solution (the final insulin concentration was 3.33 mg/mL). These suspensions were initially at room temperature ~ 25°C. The insulin/polymer suspension was pumped into the preheated chromatography column. Then incubated for 30 minutes at 90 °C. The temperature was ramped down to 25°C over 1.5 hours. The suspension was pumped from the column out into a collection vessel. Deionized water was added.

Microsphere Washing Procedure: The microsphere suspension was diluted with approximately an equal volume of deionized water in order to reduce the viscosity of the suspension. Using 50 mL polypropylene centrifuge tubes, the microsphere suspension was spun at 3500 x g for 15 minutes. The supernatant was carefully decanted from the pellet. The pellet in each tube was resuspended with deionized water equivalent to the original volume in the tube and vortexed until all of the pellet has been completely resuspended. The suspension was centrifuged at 3000 x g for 15 minutes. The supernatant was carefully decanted from the pellet. The pellet in each tube was resuspended with deionized water equivalent to the original volume in the tub and vortexed until all of the pellet was completely resuspended. The suspension was centrifuged at 3000 x g for 15 minutes. The supernatant was carefully decanted from the pellet. The pellet in each tube was resuspended with deionized water equivalent to 2/3 the volume of the tube and then vortexed until all of the pellet has been completely resuspended. The suspension was homogenized with the IKA Homogenizer at speed setting 6 for 2 minutes then centrifuged at 3000 x g for 15 minutes. The supernatant was carefully decanted from the pellet. The pellet in each tube was resuspended with a minimum volume of deionized water and vortexed until all of the pellet was completely resuspended. The suspension was homogenized with the IKA Homogenizer at speed setting 6 for 2 minutes. The microspheres were transferred to an appropriate sterile vessel and diafiltration was performed to wash microspheres until all free polymer has been removed. The microsphere suspension was concentrated using the hollow fiber cartridge system prior to lyophilization. The microspheres were bulk lyophilized under sanitary conditions, and stored dry until ready for filling.

Example 4

*MDI (metered dose inhaler) containerfillingprocess.*

Under sanitary conditions, the appropriate weight of microspheres was added to a stirred pressure vessel and the pressure vessel was charged with the appropriate volume of HFA propellant. The HFA propellant may be P134a, P227, or a blend of the two, or any other propellant(s), alone or in combination, that are useful for practicing the invention, if required. While the pressure vessel was stirring the HFA, the HFA microsphere suspension was passed through a homogenization loop until a uniform, mono-disperse suspension was achieved. Using a Pamasol or similar aerosol filling line, sterile, pre-crimped, metered dose inhaler cans or vials were charged with the mono-disperse microsphere suspension.

Example 5

*DPI (dry powder inhaler) filling process.*

Dependent on the particular product, microspheres are supplied as free-flowing microspheres suitable for auger filling or other suitable powder filling technology in the capsules, blister packs, or other suitable containers. Microspheres are added with or without bulking agent. The following bulking agents are used: sodium chloride, lactose, trehalose, sucrose, and/or others that are known to those of skill in the art.

Example 6

*Determination of microsphere particle size.*

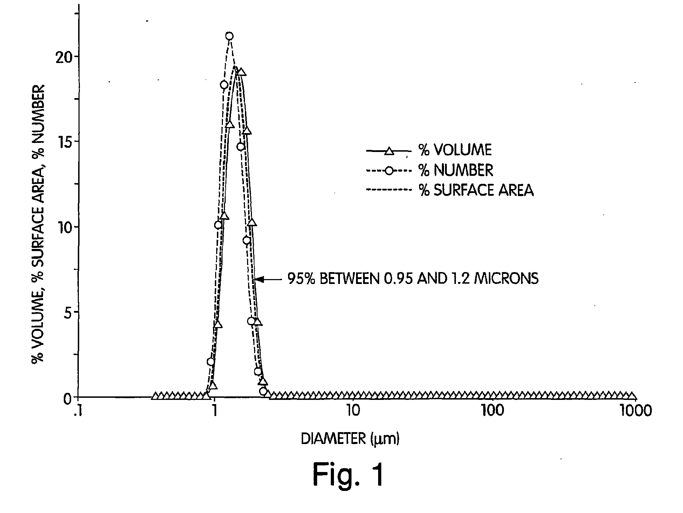

Particle size was determined by light scattering and TSI Aerosizer measurements. The insulin microspheres were mono-dispensed and are approximately 1-1.5 microns in diameter. The results typically show a homogeneous distribution of microspheres with 95% being between 0.95 and 1.20 microns in diameter by number, surface area and volume (Figure 1). The aerodynamic diameter has been shown to be 1.47 microns in diameter as seen in Figure 2.

Example 7

*In vitro Andersen cascade impaction studies.*

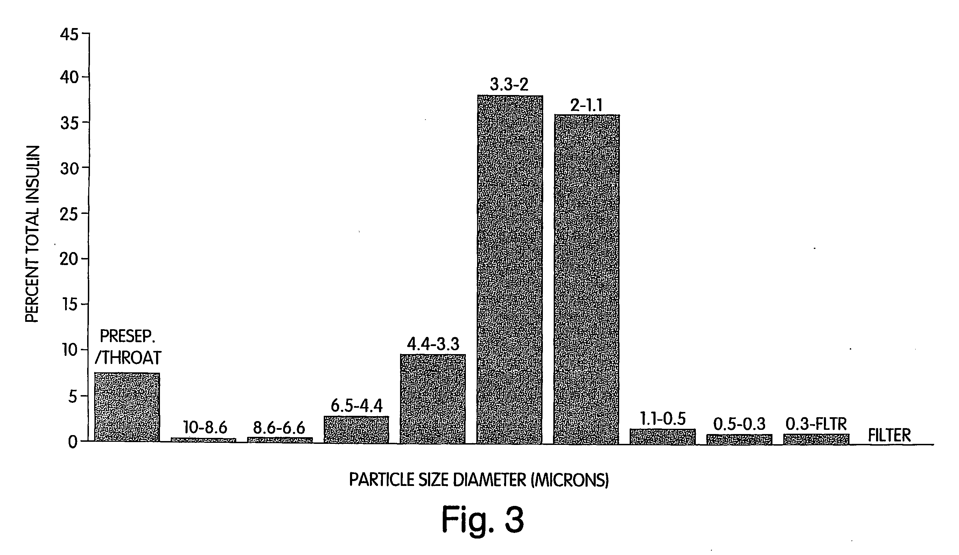

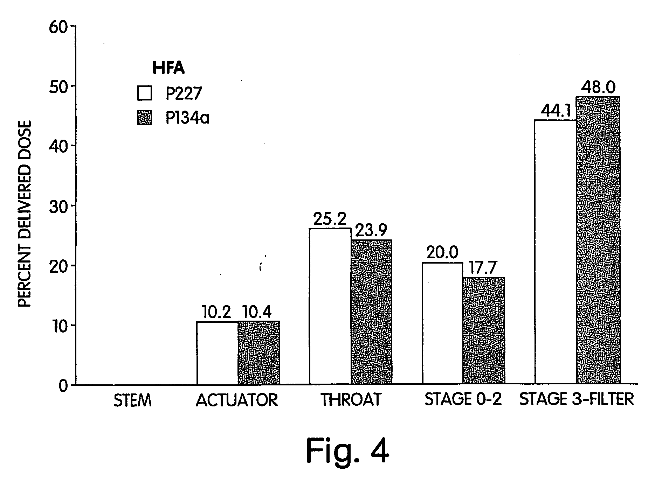

Studies with Insulin microspheres showed that a high "fine particle fraction" (FPF) of microspheres was delivered from Dry Powder Inhalers (>50-60%) (Figure 3) or from HFA (approximately 40%) (Figure 4). These represent the fractions of particle sizes that one would expect to penetrate the deep lung. These fine particle fractions are extremely high for even low molecular weight compounds. They have likely not been observed before for any protein drug delivered from a MDI.

"Fine particle fraction" (FPF) is a term of art that refers to the total amount of the drug deposited on the stages in the Andersen cascade impaction studies, within an appropriate particle size range for the drug being tested, divided by the amount total drug delivered from the mouthpiece of the inhaler into the impactor. The FPF for an MDI and a particle which is less than or equal to 4.7 $\mu$m; the FPF for a DPI and a particle which is less than or equal to 4.4 $\mu$m:

Dry Powder Inhaler: for DPI (60 lpm) a particle size range of $\leq$ 4.4 $\mu$m
Dry Powder Fine Particle Fraction (4.4) is defined as the percentage of the sum of the mass of particles less than or equal to 4.4 microns in diameter divided by the total emitted dose from the device and the mouthpiece of the device.

$$FPF = \frac{\sum Particle\ Mass \leq 4.4 \mu m}{\sum particle\ mass\ in\ all\ the\ stages\ plus\ mouthpiece} \times 100$$

Metered Dose Inhaler: for MDI (28.3 lpm) a particle size range $\leq$ 4.7 $\mu$m
Metered Dose Inhaler Fine Particle Fraction (4.7) is defined as the percentage of the sum of the mass of particles less than or equal to 4.7 microns in diameter divided by the total emitted dose from the device and the mouthpiece of the device.

$$FPF = \frac{\sum Particle\ Mass \leq 4.7 \mu m}{\sum particle\ mass\ in\ all\ the\ stages\ plus\ mouthpiece} \times 100$$

According to convention, the FPF is expressed as a percentage.

*Geometric Standard Deviation (GSD).*

A graph of cumulative percent less than the size range verses the effective cutoff diameter is plotted. From this the diameter at 84.13% and 15.37% are determined. The
GSD is calculated as:

$$GSD = (diameter\ 84.13\%/diameter\ 15.87\%)^{1/2}$$

*Mass Median Aerodynamic Diameter (MMAD).*

$$MMAD = Particle\ diameter\ at\ 50\%\ from\ the\ graph\ above$$

Stage Number – F/ Σ Drug on the stages.

Example 8

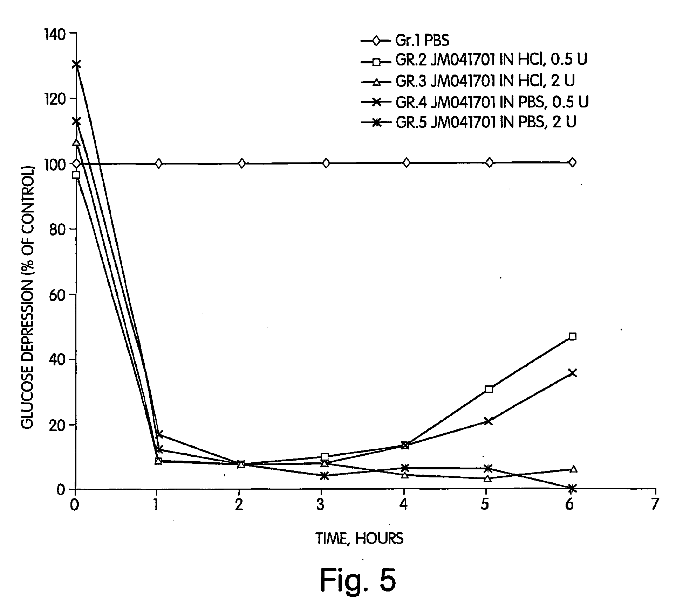

The biological activity of the insulin in Insulin microspheres was demonstrated by injecting the Insulin microspheres suspended in aqueous solutions. Figure 5 compares the blood glucose depression in normal Fisher rats after insulin injection. The results are expressed as compared to the blood glucose of control rats who received an injection of phosphate buffered saline (PBS) only. Figure 5 shows that the control animals maintained normal blood glucose concentrations over the 5 hour experiment. Microspheres that were dissolved in HCl (GR.2 and GR.3) depressed blood glucose patterns in a manner similar to intact Insulin microspheres suspended in PBS at the 0.5 Unit (U) dose and at the 2 U dose (GR.4 and GR.5).

Example 9

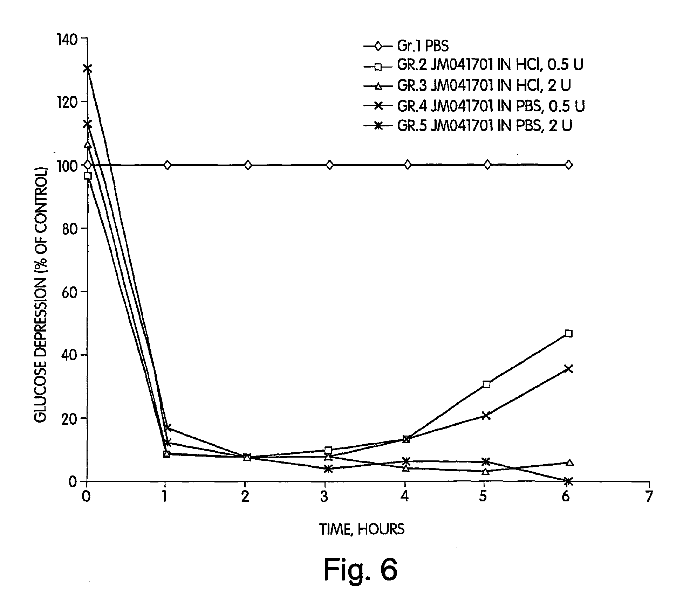

Insulin microspheres were delivered as a solution and as microspheres by directly instilling these formulations intratracheally into the lungs of Fisher rats. Figure 6 shows that a similar glucose depression pattern was observed for both the insulin solution as well as the insulin microspheres delivered as a suspension (MS YQ051401).

Example 10

*Metered Dose Inhaler Studies.*

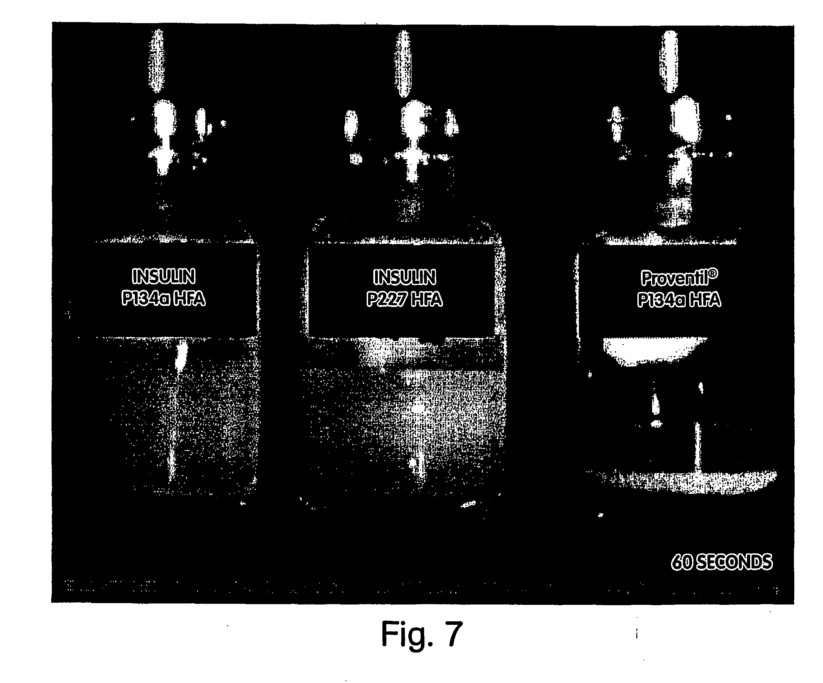

The Insulin microspheres formulated by the techniques described in the application were then formulated into CFC free propellants for use in Metered Dose Inhalers (MDIs).
Insulin microspheres were added to HFA P134a at several concentrations ranging from 2mg/ml to 10mg/d1. The suspension of insulin microspheres was compared to commercially available Proventil albuterrol in HFA P134a.
Approximately 20 mg of insulin was weighed into a 20 mL glass vial which was sealed with a valve suitable for dispensing hydrofluoroalkane (HFA) propellants P134a and P227. The addition of the HFAs resulted in the formation of a stable suspension of Insulin microspheres in HFA P134a and HFA P227 as seen in Figure 7. The Reference Vial contains commercially marketed and FDA-approved Proventil albuterol in HFA P134a. After 60 seconds the Reference Vial completely precipitated to the bottom of the vial. Those skilled in the art will recognize that this represents a source of dose irreproducibility for patients being treated with non-homogeneous suspensions. In contrast, the two vials containing insulin suspended in HFA P134a and HFA P227 remained stable homogeneous suspensions for several minutes. This represents an important property for the dispensing of reproducible dosages of drugs such as insulin from HFA propellants. Stability of insulin microspheres in HFA P134a as assessed by glucose depression *in vivo* is demonstrated in Figure 5. Bioactivity of MDA formulation at 4 months was the same as at the time 0.

Example 11

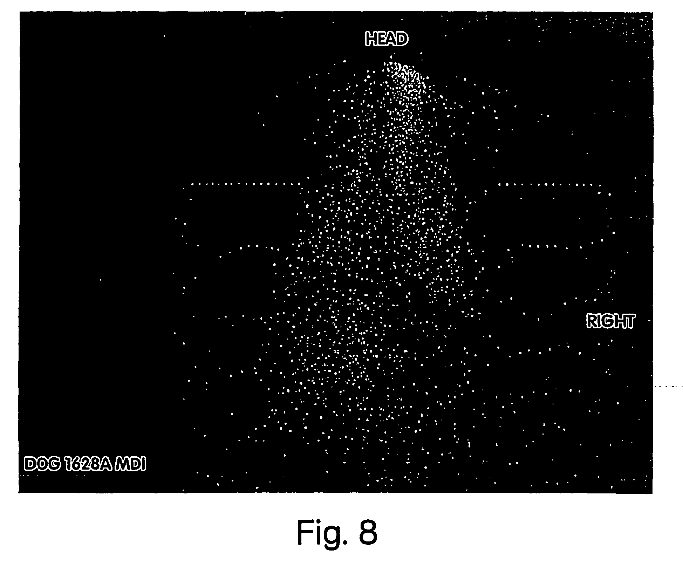

Insulin microspheres were labeled with the Tc-99m radioactive isotope. The Tc-99m insulin was then delivered to the lung of a beagle dog. A gamma camera was used to visualize the distribution of the Tc-99m labeled insulin in the dog lung. Figure 8 shows the homogeneous distribution of the Insulin microspheres throughout the lung of the lung. This indicates delivery of the microspheres to the lung.

Example 12

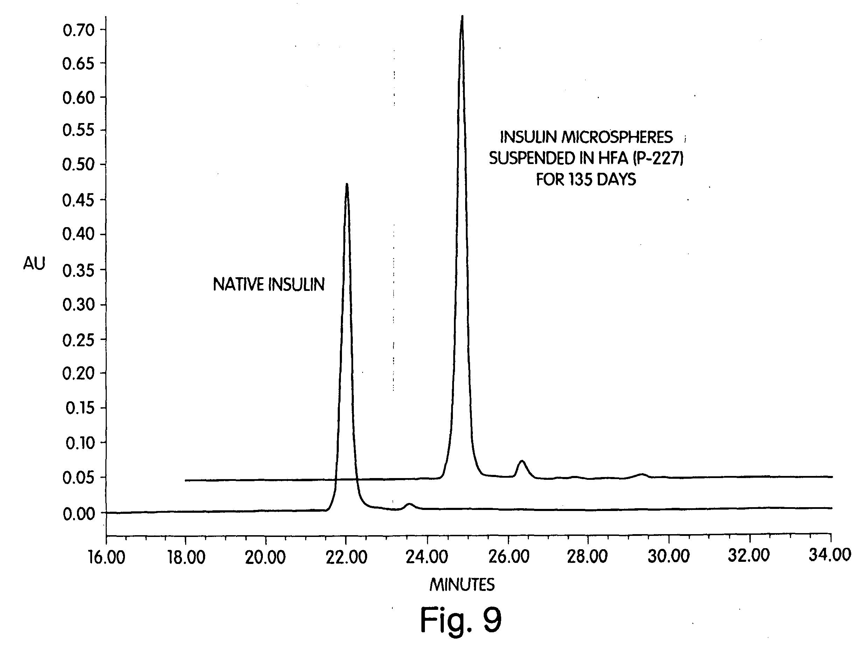

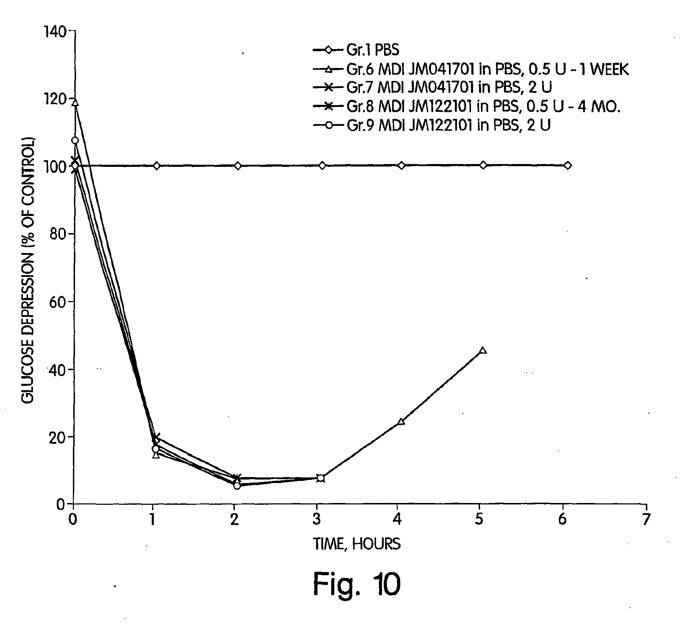

The biochemical integrity and stability of Insulin microspheres suspended in HFA P227 propellant for 135 days was shown by HPLC in Figure 9. Figure 10 shows the biological activity of Insulin microspheres stored in HFA for 7 days and 130 days. The insulin was expelled from the MDI device, and resuspended in PBS, and assayed for insulin quantity. Then, 0.5 U and 2 U of each storage time period was instilled intratracheally into Fisher rats. Figure 10 demonstrates the maintenance of biological activity *in vivo.*

Example 13

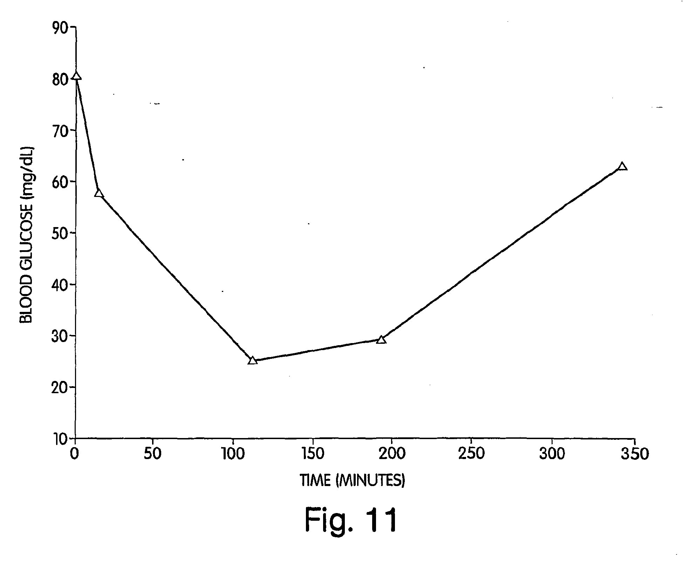

The administration of the biologically active microspheres was demonstrated in dogs. Beagle dogs were anesthetized and placed in an iron lung. Respiration rate was maintained at 75% of the pre-anesthetic breathing rate. Five mg of Insulin microspheres were delivered to the dog using an Aerolizer Dry Powder Inhaler. Figure 11 shows that a significant reduction in blood glucose was observed within 10 to 15 minutes after the pulmonary administration of the insulin. Hypoglycemic glucose concentration were maintained for over 3 hours before the administration of an oral carbohydrate feeding to the dog.

Example 14

Six MDIs were submitted for 25°C stability. After an initial conditioning time of five days at room temperature, upside-down, the samples were assayed by Andersen cascade impactor and DUSA (Dose Unit Sampling Apparatus) at 28 1pm. The DUSA experiments were done in order to determine how much of the expected delivered dose was actually delivered by the device. The data after one month of storage showed that the Andersen results exhibited a majority of the insulin microspheres that were assayed in the 1 to 3 micron sized stages.

The DUSA results showed that the recovered dose at the initial time point was $117 \pm 4.7\%$ and at one month the recovered dose was measured to be 106% of the expected dose.

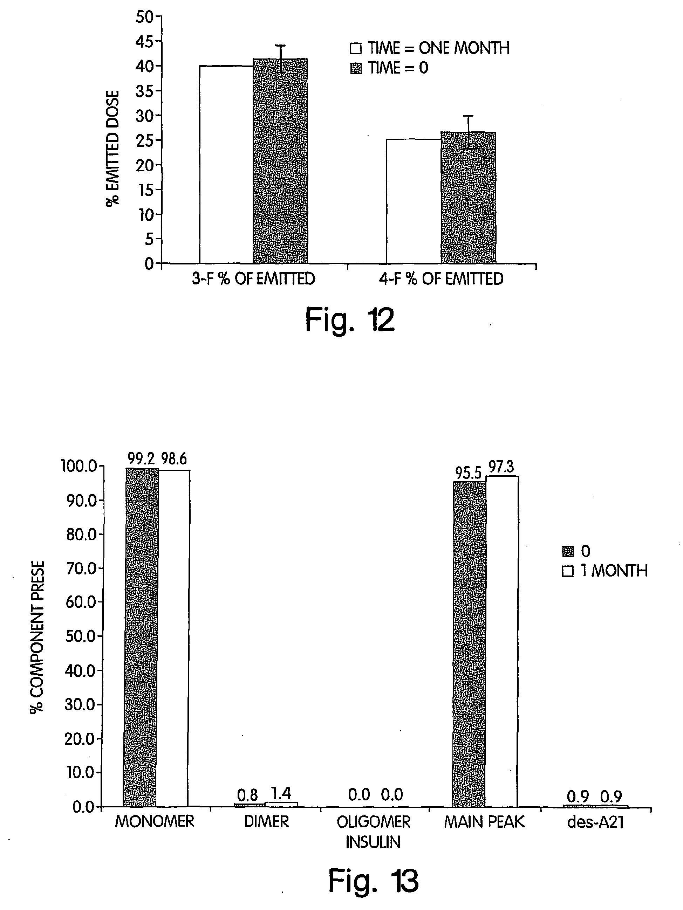

Figure 12 shows that after one month storage of the Insulin microspheres in HFA P134a, the insulin microspheres deposited in a similar fashion on stages 3 to filter and 4 to filter on the Andersen Cascade Impactor device. This indicates that the aerodynamic properties of the Insulin microspheres appear to remain stable after 1 month storage in HFA. The initial time point is the mean of 6 vials and the one month time point is the value from a single vial.

Example 15

Formulating the microspheres so that they remain biochemically stable in MDI type devices and propellants is a critical component of a MDI based insulin delivery system. The results comparing the biochemical stability of the Insulin microspheres stored in HFA for 1 month showed that the insulin monomer, dimer and oligomer were comparable as well as the main peak and desamido-insulin formation after one month (Figure 13).

Preferred aspects of the invention are set out in the following numbered paragraphs of the description.

1. A composition comprising:

(a) a plurality of microparticles, said microparticles containing a protein; and
(b) a propellant;

wherein the composition has a fine particle fraction in the range of 25% to 100%.

2. The composition of paragraph 1, wherein the microparticles have a density in the range of 0.06 gm/cc to 2.5 gm/cc.

3. The composition of paragraph 2, wherein the microparticles have a density in the range of 0.6 gm/cc to 1.8 gm/cc.

4. The composition of paragraph 1, wherein the microparticles have a narrow particle size distribution.

5. The composition of paragraph 1, wherein the microparticles are microspheres.

6. The composition of paragraph 1, wherein the propellant is a hydrofluoroalkane propellant.

7. The composition of paragraph 1, wherein the microparticles have a mean diameter in the range of from about 0.1 microns to about 10.0 microns.

8. The composition of paragraph 1, wherein the microparticles have a mean diameter in the range of from about 0.1 microns to about 5.0 microns.

9. The composition of paragraph 1, wherein the microparticles have a mean diameter in the range of from about 0.1 microns to about 3.0 microns.

10. The composition of paragraph 1, further comprising a polymer.

11. The composition of paragraph 10, wherein the polymer is water soluble or soluble in a water miscible solvent.

12. The composition of paragraph 10, wherein the polymer is selected from the group consisting of carbohydrate-based polymers, polyaliphatic alcohols, poly(vinyl)polymers, polyacrylic acids, polyorganic acids, polyamino acids, polyethers, naturally occurring polymers, polyimids, polyesters, polyaldehydes, co-polymers, block co-polymers, tertpolymers, surfactants, branched polymers, cyclo-polymers, and mixtures thereof.

13. The composition of paragraph 10, wherein the polymer is selected from the group consisting of dextran, polyethylene glycol, polyvinyl pyrrolidone, copolymers of polyethylene glycol and polyvinyl pyrrolidone, polyvinyl alcohol, copolymers of polyoxyethylene and polyoxypropylene, and mixtures thereof.

14. The composition of paragraph 10, wherein the polymer is a co-polymer of polyethylene glycol and polyvinyl pyrrolidone, and a co-polymer of polyoxyethylene and polyoxypropylene.

15. The composition of paragraph 1, wherein the microparticles are microspheres comprising greater than about 90% protein by weight.

16. The composition of paragraph 1, wherein the microparticles are microspheres comprising greater than about 95% protein by weight.

17. The composition of paragraph 1, wherein the microparticles are microspheres comprising greater than about 99% by weight.

18. The composition of paragraph 1, wherein the protein is selected from the group consisting of: leuprolide acetate, luteinizing hormone releasing hormone (LHRH), (D-Tryp6)-LHRH, nafarelin acetate, insulin, sodium insulin, zinc insulin, proinsulin, C-peptide of insulin of insulin, a mixture of insulin and C-peptide of insulin, hybrid insulin cocrystals, protamine, lysozyme, alpha-lactalbumin, basic fibroblast growth factor (bFGF), beta-lactoglobulin, trypsin, carbonic anhydrase, ovalbumin, bovine serum albumin (BSA), human serum albumin (HSA), phosphorylase b, alkaline phosphatase, beta-galactosidase, IgG, fibrinogen, poly-L-lysine, IgM, DNA, desmopressin acetate™, growth hormone releasing factor (GHRF), somatostatin, antide, Factor VIII, G-CSF/GM-CSF, human growth hormone (hGH), beta interferon, antithrombin III, alpha interferon, alpha interferon 2b, parathyroid hormone, and calcitonin.

19. The composition of paragraph 1, wherein the propellant is HFA P134a.

20. The composition of paragraph 1, wherein the propellant is HFA P227.

21. The composition of paragraph 1, wherein the composition does not comprise a surfactant.

22. The composition of paragraph 1, wherein the fine particle fraction is at least 40%.

23. The composition of paragraph 1, wherein the microparticles are in suspension.

24. The composition of paragraph 23, wherein the microparticles remain in suspension for at least 10 seconds following agitation.

25. The composition of paragraph 1, wherein the microparticles further comprise a therapeutic molecule.

26. The composition of paragraph 25, wherein the therapeutic molecule is selected from the group consisting of: albuterol, fluticazone, ipratropium bromide, beclamethasone, and other beta-agonists and steroids.

27. The composition of paragraph 25, wherein the therapeutic molecule is selected from the group consisting of: betaxolol™, diclofenac™, doxorubicin, and rifampin™.

28. The composition of paragraph 1, wherein the microparticles comprise a carbohydrate-based polymer.

29. The composition of paragraph 28, wherein the carbohydrate-based polymer comprises hetastarch.

30. The composition of paragraph 28, wherein the carbohydrate-based polymer comprises dextran sulphate.

31. A composition comprising:

a plurality of microparticles, said microparticles containing a protein; and
a propellant;

wherein the composition does not comprise a surfactant.

32. The composition of paragraph 31, wherein the microparticles remain in suspension for at least 10 seconds following agitation.

33. The composition of paragraph 31, wherein the composition has a fine particle fraction in the range of 25% to 100%.

34. The composition of paragraph 31, wherein the microparticles have a density in the range of 0.6 gm/cc to 2.5 gm/cc.

35. The composition of paragraph 31, wherein the microparticles have a density in the range of 0.6 gm/cc to 1.8 gm/cc.

36. The composition of paragraph 31, wherein the microparticles have a narrow particle size distribution.

37. The composition of paragraph 31, wherein the microparticles are microspheres.

38. The composition of paragraph 31, wherein the propellant is a hydrofluoroalkane propellant.

39. The composition of paragraph 31, wherein the microparticles have a mean diameter in the range of from about 0.1 microns to about 10.0 microns.

40. The composition of paragraph 31, wherein the microparticles have a mean diameter in the range of from about 0.1 microns to about 5.0 microns.

41. The composition of paragraph 31, wherein the microparticles have a mean diameter in the range of from about 0.1 microns to about 3.0 microns.

42. The composition of paragraph 31, further comprising a polymer.

43. The composition of paragraph 42, wherein the polymer is water soluble or soluble in a water miscible solvent.

44. The composition of paragraph 42, wherein the polymer is selected from the group consisting of carbohydrate-based polymers, polyaliphatic alcohols, poly(vinyl) polymers, polyacrylic acids, polyorganic acids, polyamino acids, polyethers, naturally occurring polymers, polyimids, polyesters, polyaldehydes, co-polymers, block co-polymers, tertpolymers, branched polymers, cyclo-polymers, and mixtures thereof.

45. The composition of paragraph 42, wherein the polymer is selected from the group consisting of dextran, polyethylene glycol, polyvinyl pyrrolidone, copolymers of polyethylene glycol and polyvinyl pyrrolidone, polyvinyl alcohol, copolymers of polyoxyethylene and polyoxypropylene, and mixtures thereof.

46. The composition of paragraph 42, wherein the polymer is a co-polymer of polyethylene glycol and polyvinyl pyrrolidone, and a co-polymer of polyoxyethylene and polyoxypropylene.

47. The composition of paragraph 42, wherein the microparticles are microspheres comprising greater than about 90% protein by weight.

48. The composition of paragraph 31, wherein the microparticles are microspheres comprising greater than about 95% protein by weight.

49. The composition of paragraph 31, wherein the microparticles are microspheres comprising greater than about 99% protein by weight.

50. The composition of paragraph 31, wherein the protein is selected from the group consisting of: leuprolide acetate, luteinizing hormone releasing hormone (LHRH), (D-Tryp6)-LHRH, nafarelin acetate, insulin, sodium insulin, zinc insulin, proinsulin, C-peptide of insulin, a mixture of insulin and C-peptide of insulin, hybrid insulin cocrystals, protamine, lysozyme, alpha-lactalbumin, basic fibroblast growth factor (bFGF), beta-lactoglobulin, trypsin, carbonic anhydrase, ovalbumin, bovine serum albumin (BSA), human serum albumin (HSA), phosphorylase b, alkaline phosphatase, beta-galactosidase, IgG, fibrinogen, poly-L-lysine, IgM, DNA, desmopressin acetate™, growth hormone releasing factor (GHRF), somatostatin, antide, Factor VIII, G-CSF/GM-CSF, human growth hormone (hGH), beta interferon, antithrombin III, alpha interferon, alpha interferon 2b, parathyroid hormone, and calcitronin.

51. The composition of paragraph 31, wherein the propellant is HFA P134a.

52. The composition of paragraph 31, wherein the propellant is HFA P227.

53. The composition of paragraph 31, wherein the fine particle fraction is at least 40%.

54. The composition of paragraph 31, wherein the microparticles are in suspension.

55. The composition of paragraph 54, wherein the microparticles remain in suspension for at least 10 seconds following agitation.

56. The composition of paragraph 31, wherein the microparticles further comprise a therapeutic molecule.

57. The composition of paragraph 56, wherein the therapeutic molecule is selected from the group consisting of albuterol, fluticazone, ipratropium bromide, beclamethasone, and other beta-agonists and steroids.

58. The composition of paragraph 56, wherein the therapeutic molecule is selected from the group consisting of: betaxolol™, diclofenac™, doxorubicin and rifampin™.

59. The composition of paragraph 31, wherein the microparticles comprise a carbohydrate-based polymer.

60. The composition of paragraph 31, wherein the carbohydrate-based polymer comprises hetastarch.

61. The composition of paragraph 59, wherein the carbohydrate-based polymer comprises dextran sulphate.

62. A method for preparing a pulmonary preparation, comprising:

selecting a propellant having a known density, $\rho_{propellant}$;
selecting a microparticle having a microparticle density $\rho_{microparticle}$ such that the ratio of $\rho_{microparticle}$ to $\rho_{propellant}$ is in the range of .05 to 30; and
contacting a plurality of the microparticles with the propellant to form the pulmonary preparation.

63. The method of paragraph 62, wherein the propellant is a hydrofluoroalkane propellant.

64. The method of paragraph 63, wherein the propellant is HFA P134a.

65. The method of paragraph 63, wherein the propellant is HFA P227.

66. The method of paragraph 62, wherein the ratio of $\rho_{microparticle}$ to $\rho_{propellant}$ is in the range of 0.5 to 3.0.

67. A method of administering a protein to the pulmonary system of a subject, comprising:

administering to the respiratory tract of a subject in need of treatment, an effective amount of the composition of any of paragraphs 1-61

68. A method of manufacture, comprising:

dispersing one, two or more therapeutic doses into a pulmonary delivery device, each of said therapeutic doses containing a therapeutically effective amount of a composition of any of paragraphs 1-61.

69. A pulmonary delivery device comprising one, two, or more therapeutic doses containing a therapeutically effective amount of a composition of any of paragraphs 1-61.

70. The device of paragraph 69, which is a metered dose inhaler.

71. A composition comprising: a package comprising:

a container having contents, said container comprising one, two or more therapeutic doses of the composition of any of paragraphs 1-61; and
instructions for using the container to deliver the contents to a pulmonary delivery device.

**Claims**

1. A composition comprising:

    (a) a plurality of microparticles, said microparticles containing a protein; and
    (b) a propellant;

wherein the composition has a fine particle fraction for a particle size of 4.7 $\mu$m or less in the range of 25% to 100%, wherein the fine particle fraction is determined using an Andersen Cascade Impactor.

2. The composition of Claim 1, wherein the microparticles have a density in the range of 0.06 gm/cc to 2.5 gm/cc.

3. The composition of Claim 2, wherein the microparticles have a density in the range of 0.6 gm/cc to 1.8 gm/cc.

4. The composition of any preceding claim, wherein the microparticles are microspheres.

5. The composition of any preceding claim, wherein the propellant is a hydrofluoroalkane propellant.

6. The composition of any preceding claim, wherein at least 90% of the microparticles have a mean aerodynamic diameter in the range of from about 0.1 microns to about 10.0 microns.

7. The composition of any preceding claim, wherein at least 95% of the microparticles have a mean aerodynamic diameter in the range of from about 0.1 microns to about 5.0 microns.

8. The composition of any preceding claim, wherein at least 95% of the microparticles have a mean aerodynamic diameter in the range of from about 0.1 microns to about 3.0 microns.

9. The composition of any preceding claim further comprising a polymer.

10. The composition of Claim 9, wherein the polymer is water soluble or soluble in a water miscible solvent.

11. The composition of Claim 9, wherein the polymer is selected from the group consisting of carbohydrate-based polymers, polyaliphatic alcohols, poly (vinyl) polymers, polyacrylic acids, polyorganic acids, polyamino acids, polyethers, naturally occurring polymers, polyimids, polyesters, polyaldehydes, co-polymers, block co-polymers, tertpolymers, surfactants, branched polymers, cyclo-polymers, and mixtures thereof.

12. The composition of Claim 9, wherein the polymer is selected from the group consisting of dextran, polyethylene glycol, polyvinyl pyrrolidone, copolymers of polyethylene glycol and polyvinyl pyrrolidone, polyvinyl alcohol, copolymers of polyoxyethylene and polyoxypropylene, and mixtures thereof.

13. The composition of Claim 9, wherein the polymer is a co-polymer of polyethylene glycol and polyvinyl pyrrolidone, and a co-polymer of polyoxyethylene and polyoxypropylene.

14. The composition of any preceding claim, wherein the microparticles are microspheres comprising greater than about 90% protein by weight.

15. The composition of any preceding claim, wherein the microparticles are microspheres comprising greater than about 95% protein by weight.

16. The composition of any preceding claim, wherein the microparticles are microspheres comprising greater than about 99% by weight.

17. The composition of any preceding claim, wherein the protein is selected from the group consisting of leuprolide acetate, luteinizing hormone releasing hormone (LHRH), (D-Tryp6)-LHRH, nafarelin acetate, insulin, sodium insulin, zinc insulin, proinsulin, C-peptide of insulin, a mixture of insulin and C-peptide of insulin, hybrid insulin cocrystals, protamine, lysozyme, alpha-lactalbumin, basic fibroblast growth factor (bFGF), beta-lactoglobulin, trypsin, carbonic anhydrase, ovalbumin, bovine serum albumin (BSA), human serum albumin (HSA), phosphorylase b, alkaline phosphatase, beta-galactosidase, IgG, fibrinogen, poly-L-lysine, IgM, DNA, desmopressin acetate, growth hormone releasing factor (GHRF), somatostatin, antide, Factor VIII, G-CSF/GM-CSF, human growth hormone (hGH), beta interferon, antithrombin III, alpha interferon, alpha interferon 2b, parathyroid hormone, and calcitonin.

18. The composition of any preceding claim, wherein the propellant is HFA P134a.

19. The composition of any preceding claim, wherein the propellant is HFA P227.

20. The composition of any preceding claim, wherein the composition does not comprise a surfactant.

21. The composition of any preceding claim, wherein the fine particle fraction is at least 40%.

22. The composition of any preceding claim, wherein the microparticles are in suspension.

23. The composition of Claim 22, wherein the microparticles remain in suspension for at least 10 seconds after agitation for microparticles in the propellant for a time sufficient to suspend the microparticles in the propellant.

24. The composition of any preceding claim, wherein the microparticles further comprise a therapeutic molecule.

25. The composition of Claim 24, wherein the therapeutic molecule is selected from the group consisting of albuterol, fluticazone, ipratropium bromide, beclamethasone, and other beta-agonists and steroids.

26. The composition of Claim 24, wherein the therapeutic molecule is selected from the group consisting of: betaxolol, diclofenac, doxorubicin, and rifampin.

27. The composition of any preceding claim, wherein the microparticles comprise a carbohydrate-based polymer.

28. The composition of Claim 27, wherein the carbohydrate-based polymer comprises hetastarch.

29. The composition of Claim 27, wherein the carbohydrate-based polymer comprises dextran sulphate.

30. The composition of Claim 1, wherein the fine particle fraction is determined using an Andersen Cascade Impactor and a metered dose inhaler.

31. The composition of Claim 1, wherein the fine particle fraction for a particle size of 4.7 $\mu$m or less is at least 50%.

32. The composition of Claim 1, wherein the fine particle fraction for a particle size of 4.7 $\mu$m or less is at least 60%.

33. The composition of Claim 1, wherein the propellant comprises a blend of propellants.

34. The composition of Claim 33, wherein the blend comprises a hydrofluoroalkane.

35. The composition of Claim 33, wherein the blend comprises HFA P134a.

36. The composition of Claim 33, wherein the blend comprises HFA P227.

37. The composition of Claim 1, wherein the propellant comprises HFA P134a and HFA P227.

38. A composition comprising:

a plurality of microparticles, said microparticles containing a protein;

wherein the composition has a fine particle fraction for a particle size of 4.4 $\mu$m or less in the range of 25% to 100%, wherein the fine particle fraction is determined using an Andersen Cascade Impactor.

39. The composition of Claim 38, wherein the microparticles have a density in the range of 0. 06 gm/cc to 2. 5gm/cc.

40. The composition of Claim 39, wherein the microparticles have a density in the range of 0. 6 gm/cc to 1. 8 gm/cc.

41. The composition of any of Claim 38 to 40, wherein the microparticles are microspheres.

42. The composition of any of Claims 38 to 41, wherein at least 90% of the microparticles have a mean aerodynamic diameter in the range of from about 0.1 microns to about 10.0 microns.

43. The composition any of Claims 38 to 42, wherein at least 95% of the microparticles have a mean aerodynamic diameter in the range of from about 0.1 microns to about 5.0 microns.

44. The composition of any of Claims 38 to 42, wherein at least 95% of the microparticles have a mean aerodynamic diameter in the range of from about 0.1 microns to about 3.0 microns.

45. The composition of any of Claims 38 to 44 further comprising a polymer.

46. The composition of Claim 45, wherein the polymer is water soluble or soluble in a water miscible solvent.

47. The composition of Claim 45, wherein the polymer is selected from the group consisting of carbohydrate-based polymers, polyaliphatic alcohols, poly (vinyl) polymers, polyacrylic acids, polyorganic acids, polyamino acids, polyethers, naturally occurring polymers, polyimids, polyesters, polyaldehydes, co-polymers, blockco-polymers, tertpolymers, surfactants, branched polymers, cyclo-polymers, and mixtures thereof.

48. The composition of Claim 45, wherein the polymer is selected from the group consisting of dextran, polyethylene glycol, polyvinyl pyrrolidone, copolymers of polyethylene glycol and polyvinyl pyrrolidone, polyvinyl alcohol, copolymers of polyoxyethylene and polyoxypropylene, and mixtures thereof.

49. The composition of Claim 45, wherein the polymer is a co-polymer of polyethylene glycol and polyvinyl pyrrolidone, and a co-polymer of polyoxyethylene and polyoxypropylene.

50. The composition of any of Claims 38 to 49, wherein the microparticles are microspheres comprising greater than about 90% protein by weight.

51. The composition of any Claims 38 to 50, wherein the microparticles are microspheres comprising greater than about 95% protein by weight.

52. The composition of any of Claims 38 to 51, wherein the microparticles are microspheres comprising greater than about 99% protein by weight.

53. The composition of any of Claims 38 to 52, wherein the protein is selected from the group consisting of leuprolide acetate, luteinizing hormone releasing hormone (LHRH), (D-Tryp6)-LHRH, nafarelin acetate, insulin, sodium insulin, zinc insulin, proinsulin, C-peptide of insulin, a mixture of insulin and C-peptide of insulin, hybrid insulin cocrystals, protamine, lysozyme, alpha-lactalbumin, basic fibroblast growth factor (bFGF), beta-lactoglobulin, trypsin, carbonic anhydrase, ovalbumin, bovineserum albumin (BSA), human serum albumin (HSA), phosphorylase b, alkaline phosphatase, beta-galactosidase, IgG, fibrinogen, poly-L-lysine, IgM, DNA, desmopressin acetate, growth hormone releasing factor(GHRF), somatostatin, antide, Factor VIII, G-CSF/GM-CSF, human growth hormone (hGH), beta interferon, antithrombinIII, alpha interferon, alpha interferon 2b, parathyroid hormone, and calcitonin.

54. The composition of any of Claims 38 to 53, wherein the composition does not comprise a surfactant.

55. The composition of any of Claims 38 to 54, wherein the fine particle fraction is at least 40%.

**56.** The composition of any of Claims 38 to 55, wherein the microparticles further comprise a therapeutic molecule.

**57.** The composition of Claim 56, wherein the therapeutic molecule is selected from the group consisting of albuterol, fluticazone, ipratropium bromide, beclamethasone, and other beta-agonists and steroids.

**58.** The composition of Claim 57, wherein the therapeutic molecule is selected from the group consisting of betaxolol, diclofenac, doxorubicin, and rifampin.

**59.** The composition of any of Claim 38 to 58, wherein the microparticles comprise a carbohydrate based polymer.

**60.** The composition of Claim 59, wherein the carbohydrate-based polymer comprises hetastarch.

**61.** The composition of Claim 59, wherein the carbohydrate-based polymer comprises dextran sulfate.

**62.** The composition of any of Claims 38 to 61, wherein the fine particle fraction is determined using an Andersen Cascade Impactor and a dry powder inhaler.

**63.** Use of a composition as claimed in any preceding claim in therapy.

**64.** A composition as claimed in any of Claims 1 to 62 for administering a protein to the respiratory tract of a subject.

**65.** A method of manufacture, comprising dispersing one, two or more therapeutic doses into a pulmonary delivery device, each of said therapeutic doses containing a therapeutically effective amount of a composition of any of Claims 1 to 62.

**66.** A pulmonary delivery device comprising one, two or more therapeutic doses containing a therapeutically effective amount of a composition of any of claims 1 to 62.

**67.** A composition comprising a package, comprising;

(a) a container having contents, said container comprising one, two or more therapeutic doses of the composition of any of claims 1 to 62; and
(b) instructions for using the container to deliver the contents to a pulmonary delivery device.

**68.** A method of preparing microparticles for use in a composition as claimed in any of claims 1 to 37, comprising mixing an aqueous solution of a protein and a polymer, said polymer being water soluble or soluble in a water-miscible solvent, and wherein the resulting microparticles have a fine particle fraction for a particle size of 4.7 $\mu$m or less in the range of 25% to 100%, wherein the fine particle fraction is determined using an Andersen Cascade Impactor.

**69.** A method according to Claim 67 further comprising the step of combining the resulting microparticles with a propellant.

**70.** A method of preparing microparticles for use in a composition as claimed in any of Claims 38 to 62, comprising mixing an aqueous solution of a protein and a polymer, said polymer being water soluble or soluble in a water-miscible solvent, and wherein the resulting microparticles have a fine particle fraction for a particle size of 4.4 $\mu$m or less in the range of 25% to 100%, wherein the fine particle fraction is determined using an Andersen Cascade Impactor.

Fig. 1

Fig. 2

## Fig. 3

PERCENT TOTAL INSULIN

PARTICLE SIZE DIAMETER (MICRONS)

PRESEP. /THROAT, 10-8.6, 8.6-6.6, 6.5-4.4, 4.4-3.3, 3.3-2, 2-1.1, 1.1-0.5, 0.5-0.3, 0.3-FLTR, FILTER

Fig. 4

Fig. 5

Fig. 6

Fig. 7

Fig. 8

Fig. 9

EP 1 944 016 A1

Fig. 10

Fig. 11

Fig. 12

Fig. 13

European Patent
Office

**EUROPEAN SEARCH REPORT**

Application Number

EP 08 07 5148

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/00215 A (ALLIANCE PHARMA ;BOT ADRIAN I (US); KABALNOV ALEXEY (US); SCHUTT E) 6 January 2000 (2000-01-06)<br><br><br>* page 24, line 3 - line 7 *<br>* page 24, line 20 *<br>* page 26, line 18 *<br>* page 31, line 22 *<br>* page 33, line 15 *<br>* page 45, line 30 *<br>* page 49, line 18 - line 19; example 22 *<br>----- | 1-3, 5-24,28, 29, 31-35, 37-55, 59,60, 62-71 | INV.<br>A61K9/00<br>A61K9/16<br>A61K38/28 |
| X | WO 96/19197 A (BAECKSTROEM KJELL ;JOHANSSON ANN (SE); ASTRA AB (SE); DAHLBAECK MA) 27 June 1996 (1996-06-27)<br><br>* examples 5,6 *<br>* page 1, line 21 - line 23 *<br>* page 4, line 26 *<br>* page 6, line 22 - line 23 *<br>----- | 1-4,6-9, 15-20, 22-24, 62-71 | |
| X | WO 94/07514 A (INHALE THERAPEUTIC SYST) 14 April 1994 (1994-04-14)<br><br><br><br><br>* page 9, line 29; claims 20,21 *<br>* page 10, line 30 - line 31 *<br>-----<br>-/-- | 1,4, 7-12, 18-21, 28,30, 31,36, 39-43, 50-52, 54,59, 61-71 | TECHNICAL FIELDS SEARCHED (IPC)<br><br>A61K |

The present search report has been drawn up for all claims

| Place of search | Date of completion of the search | Examiner |
|---|---|---|
| Munich | 6 May 2008 | Loher, Florian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                    
& : member of the same patent family, corresponding document

EPO FORM 1503 03.82 (P04C01)

European Patent
Office

# EUROPEAN SEARCH REPORT

Application Number

EP 08 07 5148

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2001/007853 A1 (DIMARCHI RICHARD DENNIS ET AL) 12 July 2001 (2001-07-12)<br><br>* paragraph [0046] - paragraph [0047] *<br>----- | 1-4,6-9, 15-20, 22-24, 62-71 | |
| X | US 3 219 533 A (MULLINS JOHN D) 23 November 1965 (1965-11-23)<br><br><br>* column 3, line 53 - line 56; example 9 *<br>* column 3, line 60 - line 70 *<br>----- | 1-4,7-9, 18, 21-27, 31, 34-36, 39-41, 50, 53-58, 62,66-71 | |
| X | BROWN ALAN R ET AL: "Tetrafluoroethane (HFC 134A) propellant-driven aerosols of proteins." PHARMACEUTICAL RESEARCH (NEW YORK), vol. 14, no. 11, November 1997 (1997-11), pages 1542-1547, XP009001798 ISSN: 0724-8741<br>* page 1542, right-hand column, paragraph 2 *<br>* page 1544, left-hand column, paragraph 1 *<br><br>* page 1545, left-hand column, paragraph 1; figures 3,4B *<br>-----<br>-/-- | 1-13, 15-24, 31-41, 51,54, 55,61-71 | TECHNICAL FIELDS SEARCHED (IPC) |

The present search report has been drawn up for all claims

| Place of search | Date of completion of the search | Examiner |
|---|---|---|
| Munich | 6 May 2008 | Loher, Florian |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P04C01)

European Patent
Office

**EUROPEAN SEARCH REPORT**

Application Number

EP 08 07 5148

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEE S-W ET AL: "DEVELOPMENT OF AN AEROSOL DOSAGE FORM CONTAINING INSULIN" JOURNAL OF PHARMACEUTICAL SCIENCES, vol. 65, no. 4, 1976, pages 567-572, XP009001721 ISSN: 0022-3549 * page 567, right-hand column, paragraph 6 * <br><br> * page 570, right-hand column, last paragraph * <br><br><br> * page 571, left-hand column, paragraph 2 * <br><br>----- | 1-4,7-9, 15-18, 22-24, 62,66-71 | |

TECHNICAL FIELDS
SEARCHED        (IPC)

The present search report has been drawn up for all claims

| Place of search | Date of completion of the search | Examiner |
|---|---|---|
| Munich | 6 May 2008 | Loher, Florian |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 08 07 5148

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2008

| Patent document cited in search report | | Publication date | Patent family member(s) | | Publication date |
|---|---|---|---|---|---|
| WO 0000215 | A | 06-01-2000 | NONE | | |
| WO 9619197 | A | 27-06-1996 | AR | 002013 A1 | 07-01-1998 |
| | | | AT | 217787 T | 15-06-2002 |
| | | | AU | 702879 B2 | 11-03-1999 |
| | | | AU | 4359196 A | 10-07-1996 |
| | | | BR | 9510501 A | 13-01-1998 |
| | | | CN | 1171046 A | 21-01-1998 |
| | | | CZ | 9701945 A3 | 15-10-1997 |
| | | | DE | 69526801 D1 | 27-06-2002 |
| | | | DE | 69526801 T2 | 09-01-2003 |
| | | | DK | 797431 T3 | 19-08-2002 |
| | | | EE | 9700137 A | 15-12-1997 |
| | | | EP | 0797431 A1 | 01-10-1997 |
| | | | ES | 2176355 T3 | 01-12-2002 |
| | | | FI | 972657 A | 19-06-1997 |
| | | | HK | 1003503 A1 | 01-11-2002 |
| | | | HU | 77701 A2 | 28-07-1998 |
| | | | IL | 116458 A | 11-01-2001 |
| | | | IS | 4496 A | 04-06-1997 |
| | | | JP | 3862178 B2 | 27-12-2006 |
| | | | JP | 10510827 T | 20-10-1998 |
| | | | JP | 2006137776 A | 01-06-2006 |
| | | | NO | 972781 A | 16-06-1997 |
| | | | NZ | 298167 A | 30-08-1999 |
| | | | PL | 320824 A1 | 10-11-1997 |
| | | | PT | 797431 T | 31-10-2002 |
| | | | RU | 2175866 C2 | 20-11-2001 |
| | | | SK | 81397 A3 | 05-11-1997 |
| | | | TR | 970135 A1 | 21-03-1997 |
| | | | TW | 398978 B | 21-07-2000 |
| | | | US | 6524557 B1 | 25-02-2003 |
| WO 9407514 | A | 14-04-1994 | AT | 220327 T | 15-07-2002 |
| | | | AU | 688283 B2 | 12-03-1998 |
| | | | AU | 5349694 A | 26-04-1994 |
| | | | BR | 9307141 A | 30-03-1999 |
| | | | CA | 2145418 A1 | 14-04-1994 |
| | | | CZ | 9500803 A3 | 15-11-1995 |
| | | | DE | 69332105 D1 | 14-08-2002 |
| | | | DE | 69332105 T2 | 06-03-2003 |
| | | | DK | 679088 T3 | 28-10-2002 |
| | | | EP | 0679088 A1 | 02-11-1995 |
| | | | ES | 2179831 T3 | 01-02-2003 |
| | | | FI | 951465 A | 10-05-1995 |
| | | | JP | 3717925 B2 | 16-11-2005 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 08 07 5148

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2008

| Patent document cited in search report | | Publication date | Patent family member(s) | | Publication date |
|---|---|---|---|---|---|
| WO 9407514 | A | | JP 8504756 T | | 21-05-1996 |
| | | | NO 951192 A | | 28-03-1995 |
| | | | NZ 257212 A | | 26-11-1996 |
| | | | PT 679088 T | | 31-12-2002 |
| | | | RU 2142815 C1 | | 20-12-1999 |
| | | | US 5607915 A | | 04-03-1997 |
| US 2001007853 | A1 | 12-07-2001 | NONE | | |
| US 3219533 | A | 23-11-1965 | BE 629985 A | | |
| | | | DE 1492015 A1 | | 06-03-1969 |
| | | | GB 1018125 A | | 26-01-1966 |
| | | | NL 289785 A | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5981719 A
- US 5849884 A
- US 6090925 A
- US 6051256 A
- US 5213812 A, Ruiz
- US 5417986 A, Reid
- US 4530840 A, Tice
- US 4897268 A, Tice
- US 5075109 A, Tice
- US 5102872 A, Singh
- US 5384133 A, Boyes
- US 5360610 A, Tice
- EP 248531 A
- US 4904479 A, Illum
- US 5149543 A, Cohen
- US 5525519 A, James E. Woiszwillo
- US 9300073 W
- WO 9314110 A
- US 5578709 A

- US 42036199 A
- US 24409800 P
- US 4008209 A
- US 4086219 A
- US 4124577 A
- US 4317815 A
- US 5110904 A
- EP 436189 A
- EP 457195 A
- EP 496452 A
- EP 528312 A
- JP 50121273 A
- US 3959247 A
- JP 521164651977 A
- US 4100152 A
- US 5514646 A, Chance
- US 255297 A, Chance
- EP 214826 A, Brange

**Non-patent literature cited in the description**

- *Nature Biotechnology,* 2002, vol. 20, 800-804
- *Endocrinology,* 1991, vol. 129, 324
- *The EMBO Journal,* 1992, vol. 11, 1867

- **BREMS et al.** *Protein Engineering,* 1992, vol. 5, 527-533
- **BRANGE et al.** *Current Opinion in Structural Biology,* 1991, vol. 1, 934-940